(19) Europäisches Patentamt
European Patent Office
Office européen des brevets

(11) **EP 2 936 155 B1**

(12) **EUROPEAN PATENT SPECIFICATION**

(45) Date of publication and mention
of the grant of the patent:
**12.12.2018 Bulletin 2018/50**

(51) Int Cl.:
**G01N 33/557** *(2006.01)*    **C12Q 1/54** *(2006.01)*
**G01N 33/66** *(2006.01)*    **G01N 21/88** *(2006.01)*
**G01N 21/27** *(2006.01)*    **G06F 19/00** *(2018.01)*

(21) Application number: **13811219.8**

(22) Date of filing: **19.12.2013**

(86) International application number:
**PCT/EP2013/077363**

(87) International publication number:
**WO 2014/096184 (26.06.2014 Gazette 2014/26)**

(54) **METHOD FOR ANALYZING A SAMPLE OF A BODY FLUID**

VERFAHREN ZUM ANALYSIEREN EINER PROBE VON KÖRPERFLÜSSIGKEIT

PROCÉDÉ POUR ANALYSER UN ÉCHANTILLON DE FLUIDE CORPOREL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2012 EP 12198443**

(43) Date of publication of application:
**28.10.2015 Bulletin 2015/44**

(73) Proprietors:
• **Roche Diabetes Care GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **RINGEMANN, Christian
68165 Mannheim (DE)**
• **PLUM, Markus
55126 Mainz (DE)**
• **PETRICH, Wolfgang
76669 Bad Schoenborn (DE)**
• **OTTENSTEIN, Timo
69115 Heidelberg (DE)**

(74) Representative: **Herzog, Fiesser & Partner
Patentanwälte PartG mbB
Dudenstrasse 46
68167 Mannheim (DE)**

(56) References cited:
**EP-A1- 1 413 883          WO-A1-99/18426
WO-A2-2006/138226      WO-A2-2011/131490
US-A- 5 420 042          US-A1- 2008 070 234**

**Description**

Field of the invention

The invention generally refers to a method for analyzing a sample of a body fluid, such as blood, interstitial fluid or other types of body fluids. The invention further relates to a computer program as well as to an evaluation device for analyzing at least one sample of a body fluid, and to a sample analysis device. Methods and devices according to the present invention specifically are applicable in the field of determining the concentration of at least one analyte in the body fluid, such as for determining a blood glucose concentration. Additionally or alternatively, however, other types of applications are feasible, such as the determination of one or more other types of analytes as well as the use of one or more other types of body fluids.

Background

In the art, a large number of devices and methods for determining one or more analytes in body fluids are known. Without restricting the scope of the present invention, in the following, mainly reference is made to the determination of blood glucose concentrations.

For performing fast and simple measurements, several types of test elements are known, which mainly are based on the use of a test substance, i.e. on the use of one or more chemical compounds or chemical mixtures adapted for performing a detection reaction for detecting the analyte. The test substance often is also referred to as the "test chemistry". For details of potential test substances, which may also be used within the present invention, reference may be made to J. Hoenes et al.: The Technology Behind Glucose Meters: Test Strips, Diabetes Technology & Therapeutics, Vol. 10, Supplement 1, 2008, S-10 to S-26. Further, reference may be made to WO 2010/094426 A1 and to WO 2010/094427 A1. Additionally or alternatively, the test substance as disclosed in WO 2007/012494 A1, WO 2009/103540 A1, WO 2011/012269 A2, WO 2011/012270 A1 or WO 2011/012271 A2 may be named, which is also referred to as the cNAD test substance. Further, reference may be made to EP 0 354 441 A2, EP 0 431 456 A1, EP 0 302 287 A2, to EP 0 547 710 A2 or to EP 1 593 434 A2. The test substances as disclosed in all these documents may also be used within the present invention. Other types of test elements and/or test substances are feasible and may be used within the present invention.

By using one or more test substances, a detection reaction may be initiated, the course of which depends on the concentration of the analyte to be determined. For deriving the concentration of the analyte, the progress of the detection reaction may be monitored by measuring and/or monitoring a time development of at least one measurement value indicating the progress of the detection reaction. This measurement value generally may comprise an arbitrary measurement value which is linked to the detection reaction, such as an optical measurement value. As an example, in many measurement setups, optical measurement values are monitored, such as a remission of a test field containing the test substance. By recording the time development of at least one measurement value, a measurement curve is provided.

A major challenge resides in a fast and, still, reliable and precise determination of the analyte concentration from the measurement curve. For this purpose, a large number of methods and devices are known in the art.

As an example, in EP 0 821 234 and in US 2002/0146835 A1, methods and devices are disclosed in which the measurement curve directly or indirectly is compared with one or more thresholds. Thus, as an example, EP 0 821 234 B1 discloses a method in which a slope of the measurement curve is determined by deriving difference values of colors and comparing these difference values with a predetermined threshold. Thereby, an end point of the detection reaction may be determined. Similarly, in US 2002/0146835 A1, an end point is determined by calculating an intermediate analyte level of the testing element at predetermined intervals and calculating a ratio value corresponding to the (n)th measurement to an (n-5)th measurement. When two consecutive ratio values are less than or equal to a predetermined value, the end point is deemed to be reached, and the final analyte level can be determined.

Further, several methods and devices using one or more fitting algorithms are known in the art, in which the measurement curve is analyzed by using one or more fit functions. Thus, in WO 2011/061257 A1, a method and a device for analyzing a body fluid are disclosed, in which a photometric measurement curve is measured. A transmission behavior of an optical transmission system is controlled by detecting measured values at two different measurement wavelengths. Further, fit functions are generated for the two measurement curves, and, by extrapolating fit curves, an offset of the measurement values is determined.

In US 2008/0087819 A1, a method for analyzing a fluid sample is disclosed, in which, again, two different wavelengths are used for deriving two measurement curves. The measurement curves are fitted by using an exponential rise with a subsequent exponential fall, by performing an appropriate fit algorithm having two different types of temporal constants.

In WO 01/25760 A1, a timing-independent method for determining a proper time for measurement of a reaction between a sample fluid and a reagent on an analyte strip is disclosed. Therein, a measurement curve of a characteristic of a matrix, to which sample fluid is applied, is periodically measured both before and after application of the sample fluid. Subsequently, a transformation is made of this measurement curve into a function which is independent in time or at most various linearly in time. The second derivative of the transformed function is then analyzed to determine when the second derivative falls below a predetermined threshold. At this point in time, the transformed function will yield the analyte concentration in the sample fluid.

In EP 1 413 883 A1, a method of reducing analysis time of end point-type reaction profiles is disclosed. For this purpose, a detection reaction is initiated, obtaining at least three measurements, at three different points in time, of a value or level of an observable associated with the detection reaction. Subsequently, an end point value for the observable is estimated from the measurements, by using an appropriate fit function.

In WO 2006/138226 A2, an arrangement and an algorithm for calculating the concentration of an analyte contained in a sample are disclosed. Therein, a color change rate of a test chemical is detected, and a hematocrit is derived from the color change rate. An appropriate correction factor indicative of the hematocrit is used for correcting a glucose concentration.

In WO 99/18426 A1, a method and a device for analyzing the concentration of an analyte in a sample, particularly the glucose content in a blood sample, is disclosed. Therein, the concentration of the analyte in the sample is determined by screening the colour reaction of a test strip over time by means of optical reflectance, wherein linear functions or polynoms are employed for evaluation purposes.

US 5 420 042 A discloses a method for the analytical determination of the concentration of a component of a medical sample, in which a reaction of the sample with reagents leads to a time-dependent change $S(t)$ in a measured quantity $S$ and the concentration $C$ correlates according to an evaluation curve $C(X)$ with an input variable $X$ derived from $S(t)$, in which the calibration curve is ambiguous for at least a portion of the possible $X$ values. In order to assign an input variable $X$ to one of the sub-sections and thereby to obtain an unambiguous correlation to a particular concentration $C$, a training run and an analysis run are performed. In the training run, a discrimination algorithm is performed at least once, in which a discriminator set is generated from measurements of $S(t)$, a score is generated in each case from the latter with a multivariate statistical technique and it is checked whether the scores can be divided into separate subsets, in which the concentrations are correctly assigned to the sub-sections of the calibration curve. The discrimination algorithm is repeated with different discriminator sets generated according to different generation methods from the measurements of $S(t)$, in order to establish an operative discrimination algorithm. In the analysis run, an analysis score is calculated from measurements of $S(t)$ according to the operative discrimination algorithm, the analysis score is compared with the boundary score and the measurement result is assigned to one of the sub-curves of the calibration curve by comparison of the analysis score with the boundary score.

WO 2011/131490 A2 discloses an improved procedure for determining the concentration or activity of an analyte in a sample. Specifically the invention provides an automated algorithm for the quality control of qPCR reactions. Plotting the fluorescence intensity of a reporter dye divided by the fluorescence intensity of a passive reference dye against the cycle number leads to a so-called sigmoid function which is characterized by a background phase, an exponential growth phase and a plateau phase. Since the fluorescence intensity as a function of cycles relates to the initial number of template molecules in the sample, qPCR curves can be used to quantify the amount of RNA or DNA fragments in the sample by determination of a so-called Cq value.

US 2008/070234 A1 discloses a method for the quantitative determination of an analyte in a sample comprising: (a) providing an analyte-specific substance which is able to undergo a reaction which generates a detectable signal when it is contacted with an analyte; (b) providing at least two calibration graphs which have been generated by reacting in each case the same analyte-specific substance with different amounts of in each case the same test analyte for in each case a predetermined reaction time; (c) contacting the analyte-specific substance with a sample which contains the analyte to be detected; (d) measuring the signal at a first predetermined reaction time for which a first calibration graph according to (b) is provided; (e) checking whether the signal measured according to (d) enables quantitative determination of the analyte with a desired accuracy; (f) (i) quantitatively determining the analyte on the basis of the signal measured according to (d) if the desired accuracy is reached, or (ii) measuring the signal at a second predetermined reaction time for which a second calibration graph according to (b) is provided; (g) checking whether the signal measured according to (f(ii)) enables a quantitative determination of the analyte with a desired accuracy; and (h) (i) quantitatively determining the analyte on the basis of the signal measured according to f(ii) if the desired accuracy is reached, or (ii) continuing the determination at at least one further predetermined reaction time (corresponding to (f)(ii), (g), (h)(i)).

While significantly improving reliability and reproducibility of analyte detection methods, the methods known in the art still may be improved in various ways. Thus, firstly, most of the fitting algorithms as known in the art are rather complicated and involve a high consumption of electrical power, hardware and software resources and evaluation time. Specifically when using hand-held devices, these aspects may lead to significant disadvantages.

Further, most of the methods and devices known in the art are susceptible to irritations and malfunctions, such as offsets, jitter or discontinuities in the measurement curves. These disturbances and artifacts, which may be due to various boundary conditions of the sample of the body fluid itself, the measurement conditions and the measurement device may impede an analytical evaluation and, in a worst case, may lead to imprecise measurement results.

Specifically, most of the methods and devices known in the art are not suited to take into account the fact that the detection reaction itself may be influenced by one or more disturbances other than the concentration of the analyte itself. Thus, specifically, in many types of test elements, a concentration of particulate components in the body fluid may have a significant impact on the measurement results. As an example, the concentration of cellular components, such as the so-called hematocrit, is known to have an influence on the analyte concentration as determined by standard test elements, such as glucose test strips. This influence may be due to the fact that sample propagation properties as well as diffusion processes are significantly altered by the presence of particulate components such as blood cells. Besides the hematocrit, other disturbance variables are known, such as the temperature of the sample and/or the measurement system. As mentioned above, methods and devices known in the art typically are not suited to take into account these disturbances when evaluating measurement curves for the purpose of determining the analyte concentration.

Problem to be solved

It is therefore an object of the present invention to provide methods and devices which at least partially overcome the disadvantages and challenges of known methods and devices. Specifically, methods and devices shall be disclosed which are suited to determine the concentration of one or more analytes in a body fluid such as blood in a simple and, still, reliable fashion, taking into account disturbances which may have an impact on a detection reaction.

Summary of the invention

This problem is solved by a method and a device for analyzing at least one sample of a body fluid, with the features of the independent claims. Preferred embodiments, which might be realized in an isolated fashion or in an arbitrary combination are listed in the dependent claims.

As used in the following, the terms "have", "comprise" or "include" or any grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

In a first aspect of the present invention, a computer-implemented method for determining a concentration c of one or more analytes in at least one sample of a body fluid is disclosed. The method comprises the following method steps. These method steps preferably are performed in the given order. However, other orders of the method steps are feasible. Further, one or more or even all of the method steps may be performed repeatedly, by repeating one of the method steps, more than one of the method steps or even all of the method steps once, twice or even more than twice. Further, two or more of the method steps may overlap in time, by performing two or more of these method steps at least partially simultaneously. As will further be outlined in detail below, one of the method steps, a plurality of the method steps or even all of the method steps may be performed by using a data processing device such as a computer, preferably a microcomputer and/or an application-specific integrated circuit (ASIC).

The method steps are as follows:

a) recording a plurality of measurement values by monitoring a time development of at least one measurement value indicating a progress of a detection reaction of at least one test substance and the sample of the body fluid, and providing at least one measurement curve F(t) which contains the measurement values, wherein at least an evaluation part of the measurement curve has an exponential characteristic, wherein the measurement values contained in the measurement curve are acquired at differing points in time, wherein the detection reaction is known to be influenced by a concentration c of an analyte to be detected in the body fluid and at least one disturbance variable Y, each disturbance variable characterizing at least one of a state of the sample of the body fluid and a condition of the detection reaction;

b) determining an end value of the measurement curve, wherein the end value forms a first variable $x_1$, wherein the end value is determined from at least one measurement value of the measurement curve, for example, by monitoring or evaluating a slope or a change in the measurement curve, and, once the slope or change reaches a predetermined threshold, by determining an end point of the detection reaction, and by choosing some or more of the measurement values acquired at or after this end point as the end value and/or by deriving the end value by combining the measurement values, such as by forming a mean end value;

c) deriving at least one fit parameter from the measurement curve by taking into account the exponential characteristic of at least the evaluation part of the measurement curve, wherein the fit parameter forms at least one second variable $x_2$;

d) deriving the concentration c of the analyte by using at least one multivariate evaluation algorithm, the multivariate evaluation algorithm being adapted to combine the first variable $x_1$ provided b step b) and the second variable $x_2$ provided by step c) into a vector $(x_1, x_2)$, wherein a weighted average of the concentration c is derived from a result vector (c, Y) of at least two procedures of the method and thereby provides the determined concentration, each procedure providing a result vector (c, Y) via a matrix transformation from the vector $(x_1, x_2)$, comprising different $x_1$ and $x_2$ values for each procedure, wherein c is the concentration of one chosen analyte, each procedure being based on a variation of one type of disturbance variable Y.

Preferably, the body fluid is selected from the group consisting of blood (such as whole blood) and interstitial fluid. However, generally, one or more other types of body fluids may be used, such as urine and/or saliva.

The analyte generally may comprise an arbitrary analyte which may be present in the body fluid. Specifically, the analyte may be a metabolite and/or may be an analyte which may take part in the metabolism of a human or an animal. Preferably, the analyte may be or may comprise glucose. However, additionally or alternatively, other types of analytes may be detected, such as lactate and/or triglycerides.

As used herein, the term "measurement value" generally refers to a quantifiable measurement result Ri, recorded by an arbitrary measurement method based on at least one of a physical, chemical and biological measurement principle. The type of measurement values may strongly depend on the type of detection reaction, as will be explained in further detail below. Thus, by using the measurement method, at least one measurement value may be determined which is known to be influenced by a detection reaction of the test substance. This measurement value preferably may be or may comprise at least one of an electrical measurement value and an optical measurement value, preferably an optical measurement value. Thus, as an example, the test substance may be part of a test field or a test area of a test element, such as a test strip. The measurement value may be an optical characteristic of the test substance, specifically the test field, such as a color and/or a photometric measurement value such as a remission value, as known in the art. The measurement value may generally be determined by using at least one detector, such as at least one optical detector. The detector preferably may comprise at least one light-sensitive element adapted to determine an intensity of light reflected by and/or emitted from the test substance, such as a test field of a test element comprising the test substance. The detector may further comprise one or more light sources for illuminating the test substance, such as for illuminating the test field. However, additionally or alternatively, other measurement principles for determining the measurement value are feasible.

As further used herein, the term "recording" refers to acquiring at least one measurement value of a sample of a body fluid, in particular, by applying at least one of a physical, chemical and biological measurement principle, preferably by employing an optical measurement principle. The recording of the measurement value may preferentially be performed in form of a spot measurement, i.e. a measurement technique wherein the measurement value may be taken within a single small area, also denoted as *spot,* particularly in order to acquire an integral value over an entire region and/or a representative value of the entire region where the measurement could be reasonably performed. In addition, the recording of the measurement value in the sample of the body fluid may particularly be performed in form of a measurement, *in vitro,* which means that the sample of the body fluid may be isolated from the body and, thus, separated from its common biological surroundings, i.e. the recording may be performed in an extra-corporal manner with respect to the body from which the sample may be taken. In a preferred embodiment, a generating of the sample by isolating the body fluid from the related body may take place prior to the recording of the at least one measurement value. However, in an alternative embodiment, the generating of the sample may be performed as a part of the present method for analyzing the sample of the body fluid, whereby the generating of the sample may, however, involve only a minor puncturing of the skin of the body, preferably at a peripheral part of the body, such as the finger tip or the ear lobe.

As further used herein, the term "measurement curve", also referred to as F(t), refers to the overall amount of data characterizing the time development or time sequence of the detection reaction. The measurement curve contains a plurality of measurement values as discussed above, recorded at different points in time. The measurement curve optionally and/or additionally may contain the respective measurement times ti of the measurement values Ri, such as by containing data pairs (Ri, ti) and/or (ti, Ri(ti)). As will be outlined in further detail below, the original measurement curve may further be replaced by a first order or higher order derivative which, then, forms a "new" measurement curve. In the following, both the option of using the original measurement curve and the option of using the new measurement curve are comprised when reference is made to the term "measurement curve".

As further used herein, the expression "monitoring" generally refers to the process of acquiring and, optionally, storing a plurality of measurement values acquired at different points in time. Thus, the monitoring simply may comprise an acquisition of electronic measurement values in conjunction with their respective times of measurement and/or acquisition. The monitoring may further optionally comprise any type of a preprocessing, processing or evaluation of the measurement curve, such as a filtering and/or a smoothing.

As used herein, the term "analyzing" generally refers to the determination of at least one of the presence and the concentration of at least one constituent or component of the body fluid. Thus, generally, the analysis may be a qualitative and/or a quantitative analysis. Preferably, the analysis is a quantitative determination of the concentration of at least one component of the body fluid, also referred to as the analyte. The analyte, as outlined above, preferably may be glucose, and the body fluid preferably may be one of blood and/or interstitial fluid. However, other embodiments are feasible.

Generally, as used herein, the term "detection reaction" refers to an arbitrary type of chemical reaction of at least one test substance and the sample of the body fluid, wherein the detection reaction is adapted to generate analysis information. Preferably, the detection reaction is a chemical reaction between at least one component of the test substance which is adapted to indicate the presence and/or the concentration of the at least one analyte in the body fluid. Thus, generally, the test substance may be a chemical compound and/or a chemical mixture which is adapted to react with the at least one analyte to be detected, preferably in a highly analyte-specific fashion. The detection reaction preferably may be embodied such that the test substance reacts with the at least one analyte to be detected and, thereby, may fully or in part change by itself, may transform into another chemical species and/or may transform its surrounding in a detectable way, which may be measured, thereby deriving the plurality of measurement values and the measurement curve. The progress of the detection reaction may be indicated by at least one physical measurement value and/or a change in at least one physical measurement value, which may be used as the measurement value as outlined above. Preferably, the detection reaction is an optically detectable detection reaction, which may be optically observable, such as by using a reflection measurement and/or a transmission measurement. Other types of measurements are feasible.

Thus, as outlined above, the term "test substance" generally refers to a chemical compound or substance or a mixture of two or more chemical compounds or substances adapted for performing the above-mentioned detection reaction, preferably an analyte-specific detection reaction. Preferably, the test substance may comprise one or more enzymes adapted to react with the at least one analyte to be detected. Additionally, the test substance may comprise one or more auxiliary components, such as mediators and/or co-enzymes. For test substances which may also be used within the present invention, reference may be made to the test substances known in the art, as discussed in more detail above, such as the cNAD test substances. Further examples will be given in further detail below. Generally, with regard to potential test substances which may be used within the present invention, reference may be made to J. Hoenes et al.: The Technology Behind Glucose Meters: Test Strips, Diabetes Technology & Therapeutics, Vol. 10, Supplement 1, 2008, S-10 to S-26. Additionally or alternatively, one or more of the test substances as disclosed in WO 2010/094426 A1 and/or in WO 2010/094427 A1 may be used. Therein, specifically, reference may be made to the test substance comprising an enzyme and a stable co-enzyme which are stored in common, specifically using carbaNAD (cNAD) as a stable co-enzyme. For details of this test substance, reference may be made e.g. to WO 2010/094426 A1. However, additionally or alternatively, other types of test substances may be used.

Further, as outlined in the context of the prior art documents, the term "disturbance value Y" generally refers to a variable other than the concentration c of the analyte, which characterizes at least one of a state of the sample of the body fluid and a condition of the detection reaction, having an impact on the plurality of measurement values and/or the measurement curve. In particular, the disturbance variable Y may comprise a parameter which may be able to influence the viscosity of the body fluid. Examples of disturbance values are: a content of at least one component of the sample of the body fluid, such as a content of a particulate component, preferably a hematocrit; a temperature of the sample of the body fluid; a humidity of an ambient atmosphere surrounding the sample of the body fluid; a parameter characterizing the quality of the test substance, such as a storage time of the test substance, the conditions under which the test substance may be stored, e. g. a possible exposition to temperature and/or humidity, including fluctuations of the temperature and/or the humidity, or a possible degradation of the test substance, the test chemistry, or a component thereof, such as an enzyme, owing, for example, to an elevated temperature, a high humidity, or a volatile material being comprised within the test chemistry or within the testing device. Additionally or alternatively, other disturbances of the detection reaction, especially an influence arising from a geometry of test strips which may be engaged in determining the analyte, such as a top dosing, a capillary channel or another geometry, are known and may be characterized by the at least one disturbance variable Y.

In the context of the present invention, the term "end value" generally refers to a value of the measurement curve at a point in time the detection reaction has essentially finished, such as by at least 70 % or more, preferably by at least 80 % or by at least 90 %. Thus, the end value preferably may be an asymptotic value of the measurement curve $F(t)$, such as for high measurement times, or an estimated asymptotic value for these high measurement times, such as a best guess for the asymptotic value. As an example, the end value may be a best guess for $lim_t \to$ oo $F(t)$, even though the measurement time typically may be limited for practical reasons. As an example for determining the end value, the slope or change in the measurement curve might be monitored or evaluated, and, once the slope or change reaches a predetermined threshold, an end point of the detection reaction may be determined, and some or more of the measurement values acquired at or after this end point may be chosen as the end value and/or the end value may be derived by combining the measurement values, such as by forming a mean end value. As an example for algorithms deriving the end value, reference may be made to the above-mentioned documents EP 0 821 234 B1, US 2002/0146835 A1 or EP 1 413 883 A1. As a further example for determining the end value, the exponential characteristic of at least the evaluation part of the measurement curve may be taken into account, from which it may be concluded that the measurement curve might approach the end value in the form of a plateau, which means that the end value may be derived from any part of the measurement curve, particularly from a part of the measurement curve which may be distant from the plateau. Additionally or alternatively, other types of algorithms may be used for deriving an end value of the measurement curve.

As further used herein, the term "fit" generally refers to an algorithm in which at least one curve to be fitted is approximated by at least one model curve or fit function, thereby modeling the shape of the curve by choosing the model curve or fit function appropriately, such as by choosing one or more parameters of the model curve or fit function appropriately. As a result of the fit, one or more fit parameters may be derived which, when used in the model curve or fit function, lead to an optimum similarity of the fit function and the curve to be fitted. To determine the similarity, known algorithms may be used. For the purpose of fitting, a large number of algorithms are known in the art, such as the method of least squares regression or least squares fit, the method of trusted region or heuristic fitting methods. Consequently, the term "fit parameter" refers to one or more parameters derived by the above-mentioned fit.

As outlined above, in method step c), at least one fit parameter is derived from the measurement curve by assuming an exponential characteristic of at least one evaluation part of the measurement curve. Thus, the whole measurement curve or a part of the measurement curve, such as a part of the measurement curve starting at a predetermined point in time or at a determinable point in time after application of the sample and/or after the start of the detection reaction, may be evaluated. As used herein, the term "exponential characteristic" generally refers to a property of a curve indicating that the curve at least partially follows or resembles a function containing one or more exponential terms. Hereby, it might be taken into account that, within the method according to the present invention, a plurality of actual measurement values are recorded by using a physical monitoring of the time development of at least one real measurement value which may be used for indicating the progress of the detection reaction of the at least one test substance. Basically it may, however, not be possible to acquire actual measurement values which might be free from any error or defect. Consequently, the term "exponential characteristic" may particularly refer to a situation wherein the curve comprising the plurality of actual measurement values at least partially follows or at least partially resembles a function which comprises one or more exponential terms, wherein, however, not each single measurement value may be obliged to obey this condition. For example, whereas an accurate exponential decay curve always requires a strictly monotonically decreasing behavior of two successive values, a real measurement curve may still be considered to exhibit the necessary exponential characteristic of at least the evaluation part of the measurement curve, even though some of the actually recorded measurement values may not follow the strictly monotonically decreasing behavior.

Preferably, one or more of the following exponential functions or exponential terms may be used as fit functions:

$$F(t) = a + b \cdot exp[-\Gamma t] \qquad (1)$$

$$F(t) = a + b \cdot exp[-\Gamma t + c] \qquad (2)$$

$$F(t) = a + b \cdot exp[-(\Gamma t)^{\beta}] \qquad (3)$$

$$F(t) = a + b \cdot exp[(-\Gamma t)^{\beta} + c] \qquad (4)$$

wherein a, b, c, $\Gamma$ and $\beta$ are parameters which may be chosen, predetermined or fitted, which may be positive or negative and which may be real numbers.

As further outlined above, in step d) of the method, at least one multivariate evaluation algorithm is used for deriving the concentration c of the analyte from at least two variables, i.e. the first variable $x_1$ (end value) and the second variable $x_2$ (fit parameter). Therein, one or more first variables and one or more second variables may be used. As used herein, the term "multivariate evaluation algorithm" generally refers to a rule or set of rules for directly or indirectly deriving the concentration c of the analyte from the at least one first variable and the at least one second variable. The evaluation algorithm generally may comprise an arbitrary mathematical algorithm or arbitrary combination of algorithms for deriving the concentration from the first variable and the second variable. Thus, the multivariate evaluation algorithm may be or may comprise a one-step algorithm in which the first variable and the second variable are used as input variables for one and the same algorithm, such as by using one and the same equation having the first variable and the second variable as input variables, thereby deriving the concentration. Alternatively, the multivariate evaluation algorithm may be or may comprise multiple steps, wherein, step-by-step, two or more algorithms are successively applied, thereby finally deriving the concentration. Therein, the first variable $x_1$ and the second variable $x_2$ may be used as variables for different steps or for the same step of the multi-step evaluation algorithm.

As an example, the at least one fit parameter and the at least one end value may be used as input variables for one equation or one algorithm, thereby deriving the concentration c in one step. Alternatively, as an example, the end value may be used for deriving an estimate value or rough value of the analyte concentration, which, subsequently, is corrected by applying a correction algorithm to the estimate value or rough value, wherein the correction algorithm comprises the at least one fit parameter, and wherein the correction is performed in accordance with the at least one fit parameter.

The method as disclosed above may be modified or may be further improved in various ways. As an example, the assumption of an exponential characteristic, which may lead to an appropriate fit function, may contain an exponential function selected from the group consisting of:

- $F(t) = a + b * \exp[-\Gamma*t]$, wherein t is the time, a is an offset, b is a contrast and $\Gamma$ is a decay constant;
- $F(t) = a + b * \exp[-(\Gamma*t)^\beta]$, wherein t is the time, a is an offset, b is a contrast, $\Gamma$ is a decay constant and $\beta$ is a stretching parameter.

Therein, a, b, $\Gamma$ and t may be real numbers. By assuming one or more of these exponential characteristics, an appropriate fit function, such as one or more of the above-mentioned functions, may be chosen in method step c).

The second variable $x_2$ may be selected from the decay constant $\Gamma$ or from a quantity which may be related to the decay constant $\Gamma$. Herein, the quantity may exhibit any relationship with the decay constant $\Gamma$, whereby a relationship wherein the quantity may be proportional to the decay constant $\Gamma$ or proportional to the inverse $1/\Gamma$ of the decay constant may be preferred. However, other kinds of relationships which may be adapted to the particular circumstances may be employed. In this embodiment, a particularly significant data reduction may be achieved since the overall amount of data of the measurement curve may be reduced to the one fit parameter either being the decay constant $\Gamma$ or the quantity in relationship with the decay constant $\Gamma$. In other words: By taking into account the exponential characteristic of at least the evaluation part of the measurement curve, the decay rate $\Gamma$ and/or the quantity in relationship with the decay constant $\Gamma$ may be determined without applying a fit procedure, by simply taking two measurement values from the evaluation part of the measurement curve from which the fit parameter may be derived. Such an appreciable simplification of acquiring the fit parameter may primarily be considered as a consequence of the exponential characteristic of at least the evaluation part of the measurement curve.

In method step c), the measurement curve itself and/or an arbitrary secondary measurement curve derived from the measurement curve may be used. Both options are possible and shall be included by the scope of the present invention. Thus, the "raw" measurement curve may, before the fitting process is performed, be subject to one or more filtering algorithms. Additionally or alternatively, one or more derivatives may be formed, thereby generating a first order derivative of the measurement curve and/or a higher order derivative of the measurement curve. Therein, arbitrary means for generating the derivatives may be used. As an example, in case the measurement curve contains a plurality of measurement values acquired at a constant acquisition rate, difference values between neighboring measurement values may be formed, and the sequence of difference values formed this way may be used as a derivative of the measurement curve. Subsequent, higher order derivatives may be formed accordingly.

In a preferred embodiment of the present invention, in step c), a first order derivative $F'(t)$ or a higher order derivative $F^n(t)$ of the measurement curve is formed before deriving the fit parameter. Thus, the first order derivative $F'(t)$ or the higher order derivative $F^n(t)$ may be subject to the fit step c), thereby deriving the at least one fit parameter.

Generally, without restricting other embodiments, the measurement values preferably may be acquired at predetermined and/or determinable points in time, and/or the measurement values may be acquired at a predetermined or determinable time span after the acquisition of the previous measurement value. Thus, as one example, the time intervals between the acquisition of neighboring measurement values may be predetermined or determinable. As a preferred example, to which the invention is not restricted, the measurement values of the measurement curve are acquired equally spaced in time, i.e. at a constant acquisition rate. Thus, the measurement curve may be acquired at a constant measurement rate or measurement frequency of 10 Hz to 100 Hz. However, other embodiments of acquisition of the measurement curve are feasible.

As outlined above, by using a simplified algorithm for deriving the first order or higher order derivatives, the first order or higher order derivatives may be approximated by calculating differences between neighboring measurement values.

In a further preferred embodiment of the present disclosure, in step c), a ratio of two subsequent derivatives $F^n(t)$ and $F^{n+1}(t)$ of the measurement curve is formed, wherein the ratio forms the fit parameter or, in case a plurality of fit parameters is used, at least one of the fit parameters. Again, the derivatives $F^n(t)$ and $F^{n+1}(t)$ may be formed by using the above-mentioned approximation by using difference values of neighboring measurement values or values of the preceding derivative.

As used herein, the formation of a ratio of two subsequent derivatives $F^n(t)$ and $F^{n+1}(t)$ of the measurement curve generally may refer to a quotient of function values the two subsequent derivatives $F^n(t)$ and $F^{n+1}(t)$ at one or more specific points in time. Additionally or alternatively, a quotient of function values of the two subsequent derivatives may be generated over a specific time span or over a plurality of points in time. Thus, as an example, an average value of a quotient of the function values of the two subsequent derivatives may be formed over a predetermined time span.

Additionally or alternatively to the option of using the "raw" measurement curve and/or a first order or higher order derivative thereof, an integral may be formed over the measurement curve. Thus, in step c), an integral may be formed over the measurement curve $F(t)$ or a first order or higher order derivative of $F(t)$, the integral forming the fit parameter. As will be outlined in further detail below, the assumption of an exponential characteristic of the measurement curve may lead to the fact that the integration results in one or more highly useful fit parameters.

The process of forming an integral, also referred to as an integration, may generally comprise an arbitrary integration algorithm known to the skilled person. Preferably, since the measurement curve or a first order or higher order derivative of the measurement curve generally are composed of discrete values such as the measurement values, the process of forming the integral may include a formation of a sum over all measurement values of the measurement curve or over a predefined group of measurement values of the measurement curve, as will be outlined in further detail below. Thus, the formation of the integral generally may imply the formation of a Riemann sum or a Riemann integral. Additionally or alternatively, however, other types of algorithms adapted for forming an integral may be used.

Further preferred embodiments of the present disclosure refer to method step d) and the above-mentioned multivariate evaluation algorithm. By using the at least one first variable $x_1$ and the at least one second variable $x_2$ and by using the above-mentioned multivariate evaluation algorithm, besides the at least one concentration c of the analyte, one or more further types of information may be generated. Thus, the multivariate evaluation algorithm may be an arbitrary algorithm or combination of algorithms by which, in addition to the concentration c of the analyte, additional information, such as the at least one disturbance variable, may be generated. Thus, generally, in step d), further, the at least one disturbance variable Y may be determined. As an example, the multivariate evaluation algorithm may be or may comprise a matrix algorithm which transforms a first vector, comprising the at least one first variable $x_1$ and the at least one second variable $x_2$ into a result vector by using a linear, quadratic or higher order matrix transformation, wherein the result vector comprises the concentration c and at least one additional information, wherein, as an example, the at least one additional information comprises the at least one disturbance variable Y. As an example, besides the concentration c, the at least one hematocrit might be determined and/or the temperature of the sample of the body fluid. For this purpose, as an example, the at least one multivariate evaluation algorithm may comprise a step of transforming the vector $(x_1, x_2)$ by using a transformation matrix having coefficients $c_{ij}$, which may be determined by an arbitrary calibration algorithm. By multiplying the vector $(x_1, x_2)$ with this matrix, a result vector $(c, Y)$ might be generated. Other examples are feasible.

Further embodiments refer to the above-mentioned deriving of the end value of the measurement curve, as described in step b) of the present invention. As further used herein, the term "deriving" may comprise any procedure which may be configured for acquiring the end value of the measurement curve. Herein, a procedure which may determine the end value by using an actually recorded property of the measurement curve an deriving therefrom the desired value may be particularly preferred. Preferred examples for the actually recorded property include a slope of the measurement curve which may be compared to at least one threshold value, or a part of the measurement curve which may even be distant from the plateau formed by the end value. Alternatively, it may be feasible to determine the end value by using a model adapted to provide the end value from any known parameters otherwise related to the sample of the body fluid.

Thus, as disclosed above and as disclosed in the above-mentioned prior art, in step b), the slope of the measurement curve may be compared to the at least one threshold value for determining the measurement curve has reached the end value. As an example, the slope may be formed by a difference value between neighboring measurement values of the measurement curve, specifically in case a constant acquisition rate or measurement rate is used for acquiring the measurement values. Thus, difference values of neighboring measurement values may be formed and may be compared to at least one threshold value, for determining if the end point of the reaction has been reached. Therein, additional criteria might be added, such as a criterion indicating that at least two, at least three or at least a specific number of neighboring difference values are below or above the threshold value. For example, the threshold value may be a threshold value indicating that a change in the reflectance values per second is below 3 %, 2 % or even 1 %.

A further embodiment may alternatively or additionally used for the deriving of the end value of the measurement curve according to step b), wherein the end value forms a first variable $x_1$. This embodiment may be particularly based on the exponential characteristic of at least the evaluation part of the measurement curve. Taking the exponential characteristic of at least the evaluation part into account, it may be concluded that the measurement curve might approach the end value after a certain period of time, wherein the end value may exhibit the form of a plateau. Hereby, every measurement curve may form a same plateau value independent from the at least one disturbance variable Y. Thus, it might be possible to derive the analyte concentration independent from the at least one disturbance variable Y. As a non-limiting example, the glucose concentration may be derived from a remission curve in an optical measurement since all remission curves may form the same plateau value independent from the actual haematocrit or temperature. Moreover, the exponential characteristic may, thus, be employed to determine the plateau value by utilizing measurement values taken from a part of the measurement curve which may not necessarily bear any relation to the plateau. Already the fact that at least the evaluation part of the measurement curve may comprise an exponential shape may allow deducting information about the end value from any part of at least the evaluation part of the measurement curve.

Consequently, the end value may be derived from an earlier part of the measurement curve, wherein the earlier part may be a part of the measurement curve being distant from the plateau. As a result, the end value may be derived according to step b) as the at least one first variable $x_1$ from at least one measurement value taken from the measurement curve whereas the at least one second variable $x_2$ may be derived according to step c) from at least one fit parameter as derived from the measurement curve.

This feature may imply that it may not be necessary to acquire measurement values until the measurement curve may have reached a predefined threshold value. According to the present embodiment, it may rather be feasible to derive the end value already from the earlier part of the measurement curve, preferably from the same part of the measurement curve in which the decay constant $\Gamma$ or a quantity which may be related to the decay constant $\Gamma$ may be determined as the second variable $x_2$. Without loosing information, a lower number of actually recorded measurement values may, thus, be sufficient for determining the concentration of the analyte. On the other hand, since the accuracy of the end value may increase when the plateau value may be derived at a later part of the measurement curve, an optimum time to terminate the recording of the measurement values may be found somewhere midway through the measurement curve. Irrespective of the actually chosen time to terminate the recording of the measurement values, a saving of resources, including but not limited to measurement time, calculating efforts and/or memory space, which might be considerably, may be achieved by application of this embodiment.

Further preferred embodiments refer to the above-mentioned evaluation part of the measurement curve. As indicated above, the evaluation part generally may be an arbitrary part of the measurement curve or even the full measurement curve. As a preferred example, the evaluation part of the measurement curve is a part of the measurement curve starting at a predetermined or definable starting point after a commencement of a measurement, i.e. after an application of the sample to the test substance and/or after a start of the detection reaction. Thus, as an example, the evaluation part of the measurement curve may be a remainder of the measurement curves starting after a definable starting time span after a commencement of the measurement. The starting time span generally may be a definable or predetermined time span, such as a fixed time span of 0.5 s to 3 s, preferably 1.0 s to 2.0 s and, most preferably, 1.5 s to 1.7 s. By applying this predetermined time span, an initial phase of the measurement curve may be excluded from the evaluation, wherein the initial phase, as an example, may include a wetting period during which the test substance is wetted by the sample.

Further embodiments relate to the multivariate evaluation algorithm. As indicated above, the multivariate evaluation algorithm may be or may comprise an arbitrary one-step or multi-step evaluation algorithm which transforms the at least one first variable $x_1$ and the at least one second variable $x_2$ into the concentration c and, optionally, into additional information. As outlined above, the multivariate evaluation algorithm might comprise a linear matrix algorithm and/or a linear equation, having two or more coefficients, by which the at least one first variable $x_1$ and the at least one second variable $x_2$ are transformed into the concentration c and, optionally, into additional information, such as into the at least one disturbance variable Y. Additionally or alternatively, the multivariate evaluation algorithm may be or may comprise a non-linear equation system and/or a non-linear transformation matrix algorithm, again which, again, comprises two or more coefficients. Further, two or more evaluation algorithms may be provided, such as two or more transformation algorithms and/or two or more transformation curves. One or more of these evaluation algorithms may be chosen out of the plurality of multivariate evaluation algorithms, such as according to appropriate boundary conditions. As an example, a temperature of the environment may be measured independently, and an appropriate multivariate evaluation algorithm corresponding to the specific ambient temperature as measured may be chosen from a plurality of multivariate evaluation algorithms, thereby choosing an appropriate multivariate evaluation algorithm for the respective temperature of the sample of the body fluid.

The method according to the present disclosure may further imply the use of at least one decision tree. Thus, at least one decision tree may be employed within the method for analyzing the sample of the body fluid. As further used herein, a "decision tree" may comprise at least one decision branch which may allow selecting one out of at least two, preferably two, alternative functions based on an assessment whether a predetermined condition may be fulfilled or not. The decision branch itself may comprise an additional second-order decision branch which may allow performing one out of at least two, preferably two, further alternative functions depending on the assessment of a further predetermined condition. In addition, the second-order decision branch may comprise at least one further higher-order decision branch. In general, the predetermined condition may assess an existence of a value, a non-existence of a value, or whether a definite value falls within at least one predetermined range or not. The decision branch may, thus, offer a decision between performing or not performing a specific function or performing the specific function under a specific parameter, with a specific parameter set, or within a specific parameter range. As a non-limiting example, only such glucose values may be submitted to a correction procedure for which such a correction may be required, e.g. outside the predetermined hematocrit range. Another non-limiting example may refer to threshold values which may be applied for determining the glucose concentration in a sample, wherein the actual threshold values applied within this procedure may be selected according to a predetermined glucose concentration range.

In addition, a weighted average is employed within the method for analyzing the sample of the body fluid for taking into account the results out of at least two, preferably a multitude of, procedures based on variations of the at least one disturbance variable Y in order to derive a value for the concentration c of the analyte. Herein, the weighted average may comprise weights which may denote probabilities for each specific value of the disturbance variable Y according to a forecast model which may reflect the probability distribution of each specific value of the disturbance variable Y. As a non-limiting example, a number of glucose concentrations may, thus, be obtained, each glucose concentration for a specific value of the hematocrit within a predetermined range, and the weighted average thereof may be derived, thereby acquiring a single value for the glucose concentration. Herein, the weights may denote probabilities for each specific value of the hematocrit according to a forecast model which may reflect the probability distribution of each specific value of the hematocrit.

The multivariate evaluation algorithm generally may be determined in a preceding method step, such as by using a plurality of calibration measurements. Thus, in a simple measurement setup, a plurality of calibration samples may be provided, having well-defined and different analyte concentrations and/or having well-defined and different disturbance variables. In a simple case, the multivariate evaluation algorithm may comprise a multiplicity of coefficients, such as the coefficients of a transformation matrix, which may be determined by solving the equation system resulting from applying these coefficients to the measurement results $x_1$ and $x_2$ resulting from measurements using the calibration fluids. The skilled person immediately will recognize a number of potential calibration setups. Thus, generally, in the context of the present invention, the term "calibration measurement" may refer to an arbitrary measurement acquired by using a calibration fluid and/or acquired under known conditions, such that at least the concentration and at least one disturbance variable are known. Thus, in case the disturbance variable refers to the calibration fluid, the disturbance variable may be known via the calibration fluid itself, such as by using a calibration fluid having a predetermined hematocrit. In case the target variable refers to the measurement conditions, such as a temperature and/or specific properties of the test substance used for the measurement, the disturbance variable may be known from the circumstances of the measurement. Thus, by using one or more calibration measurements, at least one multivariate evaluation algorithm may be determined and/or a set of multivariate evaluation algorithms may be determined, and, preferably, stored in a data storage for later use by the method according to the present invention.

In further aspect of the present invention, an evaluation device for analyzing at least one sample of a body fluid as defined in the claims is disclosed. As applies to the method as disclosed in one or more of the embodiments listed above, the evaluation device preferably may be adapted for evaluating a measurement curve for the purpose of analyzing the sample of the body fluid. The evaluation device comprises at least one evaluation unit, wherein the evaluation unit is adapted to perform the method according to one or more of the embodiments disclosed above and/or according to one or more of the embodiments disclosed in further detail below. As an example, the evaluation unit may comprise one or more data processing devices, such as one or more computers and/or application-specific integrated circuits (ASICs), preferably at least one microcomputer. The at least one data processing device may comprise one or more software components adapted to run on the data processing device, the software components being adapted to perform the method according to the present invention, fully or partially, e.g. except for specific measurement steps which might be involved in the recording of the measurement values and which might be performed by one or more measurement devices connected to the processor. The measurement values, in the latter case, may be provided to the evaluation unit, as a part of the recording step. The evaluation unit, which may be or which may comprise one or more components, may preferably be adapted to perform a software algorithm implementing the above-mentioned method in one or more of the embodiments listed above and/or as disclosed in further detail below.

In a further aspect of the present invention, a sample analysis device for characterizing a sample of a body fluid as defined in the claims is disclosed. As used herein, the term "characterizing" relates to a process of determining one or more properties of the sample of the body fluid. Specifically, as will be disclosed in further detail below, the term "characterizing" refers to the fact that a concentration of at least one analyte in the body fluid may be determined. Additionally, one or more items of information regarding the sample of the body fluid may be generated, such as an information on the at least one disturbance variable.

The sample analysis device comprises at least one measuring unit for measuring a detection reaction of at least one test substance and at least one sample of a body fluid. Therein, the detection reaction is known to be influenced by a set of disturbance variables, each disturbance variable characterizing at least one of a state of the sample of the body fluid and a condition of the detection reaction. The measuring unit further is adapted for monitoring a time development of at least one measurement value indicating a progress of the detection reaction, thereby recording a measurement curve F(t) containing a plurality of the measurement values acquired at different points in time.

The at least one measuring unit, as outlined above, may comprise one or more detectors for measuring the plurality of measurement values, which, in the following, will be denoted by Ri, the plurality of measurement values forming the measurement curve F(t) and/or a part of the measurement curve. The at least one detector may be or may comprise an arbitrary element for determining the at least one measurement value, such as an optical detector and/or an electrical detector. As an example, an optical detector may be provided, having at least one light-sensitive element, such as a photodiode and/or a photocell, for measuring light reflected by the test substance, such as by a test field of a test element, the test field comprising the test substance, and/or by measuring light transmitted by the test substance. The at least one detector may further comprise one or more light sources for illuminating the test substance, such as one or more of a light-emitting diode, a laser diode or a light bulb. The measuring unit may be adapted to acquire the measurement values generated by the detector, which may be provided in an arbitrary form, such as in the form of electrical signals and/or in the form of analog and/or digital signals. The measuring unit may further be adapted for storing these measurement values and/or for transferring these measurement values to another unit of the sample analysis device, such as to a display or to an evaluation device as will be disclosed in further detail below.

The sample analysis device further comprises at least one evaluation device according to the present invention, as disclosed above or as disclosed in further detail below. The evaluation device, preferably, may be or may comprise at least one data processing device, such as at least one computer or computer network. Thus, the evaluation device may be or may comprise a microcomputer integrated into the sample analysis device and/or may be or may comprise a computer which is connected to the measuring unit by at least one interface and/or at least one data connection.

As outlined above, the test substance preferably may be part of a test element. The test element, as known in the art, may comprise one or more test fields comprising the at least one test substance, such as one or more test fields applied to a surface of a carrier element of the test element. As an example, the test element may be or may comprise one or more of a test strip, a test tape, a test disc or any other type of test element known in the art. The test element generally may contain the at least one test substance adapted to perform the detection reaction. The sample analysis device may be adapted such that the sample of the body fluid is applicable to the test element. Thus, the sample analysis device may comprise one or more receptacles for receiving the at least one test element, wherein the test element and/or the sample analysis device comprises one or more application positions and/or application mechanisms in which the sample of the body fluid may be applied to the at least one test substance.

As outlined above or as will be outlined in further detail below, the method according to the present invention is highly efficient and is adapted to generate measurement results such as the analyte concentration and, optionally, the at least one disturbance variable Y, rather quickly and, still, precisely. Thus, the present invention specifically is applicable in small, portable devices which, typically, are rather limited with regard to their hardware and software resources. Therefore, preferably, the sample analysis device may be embodied as a hand-held device. As used herein, the term hand-held device generally refers to a device which is portable by a user, such as in one hand. Typically, the hand-held device may be a device having a volume of less than 1000 cm$^3$, preferably of less than 500 cm$^3$. The weight of the hand-held device preferably is less than 1 kg, preferably less than 500 g.

The method, the computer program, the evaluation device and the sample analysis device according to the present invention provide a large number of advantages over known methods, computer programs and devices. Thus, as will be outlined in further detail below, the general concept of using a first variable $x_1$ indicating the end value of the measurement curve and, additionally, using at least one fit parameter derived by assuming an exponential characteristic of the measurement curve or at least an evaluation part thereof as at least one second variable $x_2$, allows for a multiplicity of evaluation options, which are easily implemented.

Thus, as a first option, a simple exponential function may be fitted to the measurement curve, thereby deriving at least one fit parameter, to be used as the additional, second variable $x_2$.

As a second option, a first order or higher order derivative of the measurement curve may be used and may be fitted, whereby, as is evident from equations (1) to (4) as given above, the offset of the measurement curve may be eliminated.

As a third option, as also evident from the potential fit functions indicating the exponential characteristic above, specifically when considering equation (1) given above, the option of forming a quotient of two subsequent derivatives of the fit function may provide an easy algorithm for determining the parameter Γ, which may indicate a decay rate or an increase rate of the exponential characteristic.

As a fourth option, as also evident by e.g. using one or more of the equations (1) to (4) given above, specifically equation (1), an integration from 0 to ∞ may lead to a simple, constant quotient b/Γ, wherein b is the contrast of the exponential characteristic, and Γ is the decay constant.

As a fifth option, two separate equations for the first derivative of equation (1), wherein the base line may be neglected (a = 0), may be set up for two differing threshold values, wherein the parameter Γ, which may indicate a decay rate, may be obtained from the two equations, for example, by a rearranging of the equations and a subsequent substitution. Hereby, the two differing threshold values may be particularly selected from a range from -10 %/s to -1 %/s, preferably from -5 %/s to -2 %/s.

Thus, these five options, which may be applied individually or which may be used in arbitrary combination, may lead to a simple, efficient generation of at least one fit parameter or additional variable $x_2$, which may be used for the multivariate analysis of the measurement curve.

Thus, by combining the end value and a fit parameter, an efficient and, still, precise algorithm may be provided, which, as an example, is adapted for correcting the concentration c for a current hematocrit. As will further be presented, the present method has especially proved to be particularly suited for a correction of the glucose concentration by considering the current hematocrit under which the amount of glucose may be determined when analyzing a sample of blood. By taking into account an exponential characteristic for the measurement curve or a derivative thereof, the information contained in the measurement curve, such as in a chemical kinetic remission curve, may be reduced to a few fit parameters, such as to the above-mentioned offset a, the contrast b and the decay rate $\Gamma$. The behavior of these parameters with regard to disturbance variables such as hematocrit, temperature or relative humidity may be used in order to generate a corrected concentration of the analyte and/or for correcting a raw value of the analyte concentration. Within this regard it may be explicitly mentioned that a knowledge of the disturbance variables may not be required for accurately determining the concentration of the analyte by employing the method according to the present disclosure.

Thus, in addition to the end value, one or more additional variables $x_2$, such as one or more of the fit parameters a, b, $\Gamma$, $\beta$ or any combination thereof, may be used for improving the measurement result of the determination of the analyte concentration. Thus, the assumption of an exponential characteristic of at least the evaluation part of the measurement curve or a derivative thereof may lead to a significant data reduction, since the overall amount of data of the measurement curve may be reduced to one fit parameter and/or a set of a few fit parameters. This feature may be useful to reduce the amount of memory space required for storing data and calculating parameters within the sample analysis device which might be particularly helpful for decreasing the size of a hand-held device.

By using a derivative of the measurement curve, when assuming an exponential characteristic, the offset of the measurement curve may easily be eliminated. Similarly, by assuming an exponential characteristic, the decay rate $\Gamma$ and/or the contrast b of the exponential function may be determined without using a fit, by forming the above-mentioned quotient of two subsequent derivatives of the measurement curve. Thus, the effort and the resources for performing a fit may even fully or partially be eliminated. Therewith, the costs of the evaluation device and/or of the sample analysis device may significantly be reduced. Further, the lifetime of a battery and/or another optional energy storage device of the sample analysis device may be increased significantly.

The assumption of an exponential characteristic and the use of a fit of an exponential function may also be extended, by using a "stretched" exponential function, as indicated by equation (4) above. Therein, the stretching parameter $\beta$ may be used as an additional parameter, which, in addition or as an alternative to the other parameters a, b and $\Gamma$, may be dependent on the concentration of the analyte, such as the glucose concentration, and, in addition, may depend on one or more disturbance variables, such as hematocrit, relative humidity, temperature and other disturbance variables. Thus, the stretch factor $\beta$ may be used for correcting the analyte concentration, by using the method according to the present invention. The invention further discloses and proposes a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps a) to d) as indicated above may be performed by using a computer or a computer network, preferably by using a computer program.

The invention further discloses and proposes a computer program product having program code means, in order to perform the method according to the present invention in one or more of the embodiments enclosed herein when the program is executed on a computer or computer network. Specifically, the program code means may be stored on a computer-readable data carrier.

Further, the disclosure and proposes a data carrier having a data structure stored thereon, which, after loading into a computer or computer network, such as into a working memory or main memory of the computer or computer network, may execute the method according to one or more of the embodiments disclosed herein.

The disclosure further proposes and discloses a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the disclosure proposes and discloses a modulated data signal which contains instructions readable by a computer system or computer network, for performing the method according to one or more of the embodiments disclosed herein.

Preferably, referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present disclosure further discloses:

- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing the findings of the present disclosure, the following embodiments are preferred:

Embodiment 1: A computer-implemented method for determining a concentration c of one or more analytes in at least one sample of a body fluid, thereby taking into account disturbances having an impact on a detection reaction the method comprising the following steps:

a) recording a plurality of measurement values by monitoring a time development of at least one measurement value indicating a progress of a detection reaction of at least one test substance and the sample of the body fluid, and providing at least one measurement curve $F(t)$ which contains the measurement values, wherein at least an evaluation part of the measurement curve has an exponential characteristic, wherein the measurement values contained in the measurement curve are acquired at differing points in time, wherein the detection reaction is known to be influenced by both a concentration c of an analyte to be detected in the body fluid and a set of disturbance variables Y, each disturbance variable characterizing at least one of a state of the sample of the body fluid and a condition of the detection reaction;

b) deriving an end value of the measurement curve provided in step a), wherein the end value forms a first variable $x_1$, wherein the end value is derived from at least one measurement value of the measurement curve;

c) deriving at least one fit parameter from the measurement curve provided in step a) by taking into account the exponential characteristic of at least the evaluation part of the measurement curve, wherein the fit parameter forms at least one second variable $x_2$, wherein the second variable $x_2$ is derived from at least one fit parameter from the measurement curve;

d) deriving the concentration c of the analyte by using at least one multivariate evaluation algorithm, the multivariate evaluation algorithm being adapted to combine the first variable $x_1$ provided in step b) and the second variable $x_2$ provided by step c), wherein a weighted average of results of at least two procedures based on variations of the at least one disturbance variable Y are provided in order to derive a value for the concentration c of the analyte.

Embodiment 2: The method according to the preceding embodiment, wherein the body fluid is selected from the group consisting of blood and interstitial fluid.

Embodiment 3: The method according to any one of the preceding embodiments, wherein the analyte is glucose.

Embodiment 4: The method according to any one of the preceding embodiments, wherein the test substance contains at least one enzyme, preferably GOD and/or GDH.

Embodiment 5: The method according to any one of the preceding embodiments, wherein the measurement values are optical measurement values.

Embodiment 6: The method according to the preceding embodiment, wherein the optical measurement values are detected by a reflective measurement.

Embodiment 7: The method according to any one of the two preceding embodiments, wherein the measurement values are remission values.

Embodiment 8: The method according to one of the preceding embodiments, wherein the disturbance variable Y comprises a parameter which is able to influence the viscosity of the body fluid.

Embodiment 9: The method according to one of the preceding embodiments, wherein the at least one disturbance variable is selected from the group consisting of: a particulate content of the sample, preferably a hematocrit.

Embodiment 10: The method according to any one of the preceding embodiments, wherein the exponential characteristic contains at least one exponential function selected from the group consisting of:

- $F(t) = a + b * \exp[-\Gamma t]$, wherein t is the time, a is an offset, b is a contrast and $\Gamma$ is a decay constant;
- $F(t) = a + b * \exp[-(\Gamma*t)^{\beta}]$, wherein t is the time, a is an offset, b is a contrast, $\Gamma$ is a decay constant and $\beta$ is a stretching parameter.

Embodiment 11: The method according to the preceding embodiment, wherein the second variable $x_2$ is selected from the decay constant $\Gamma$ or from a quantity which is in relationship with the decay constant $\Gamma$.

Embodiment 12: The method according to the preceding embodiment, wherein the quantity is proportional to the decay constant $\Gamma$ or proportional to the inverse $1/\Gamma$ of the decay constant.

Embodiment 13: The method according to any one of the preceding embodiments, wherein, in step c), a first order derivative $F'(t)$ or a higher order derivative $F^n(t)$ of the measurement curve is formed before deriving the fit parameter.

Embodiment 14: The method according to the preceding embodiment, wherein the measurement values of the measurement curve are acquired equally spaced in time.

Embodiment 15: The method according to the preceding embodiment, wherein the measurement curve is acquired at a constant measurement frequency of 10 Hz to 100 Hz.

Embodiment 16: The method according to any one of the two preceding embodiments, wherein the first or higher order derivative is approximated by calculating differences between neighboring measurement values.

Embodiment 17: The method according to any one of the preceding embodiments, wherein, in step c), a ratio of two subsequent derivatives $F^n(t)$ and $F^{n+1}(t)$ of the measurement curve is formed, the ratio forming the fit parameter.

Embodiment 18: The method according to any one of the preceding embodiments, wherein, in step c), an integral is formed over the measurement curve $F(t)$ or a first order or higher order derivative of $F(t)$, the integral forming the fit parameter.

Embodiment 19: The method according to any one of the preceding embodiments, wherein, in step c), the fit parameter is obtained from a comparison of a first order derivative of the measurement curve at two differing points in time.

Embodiment 20: The method according to the preceding embodiment, wherein the two differing points in time are obtained by applying two differing threshold values.

Embodiment 21: The method according to the pre-preceding embodiment, wherein at least one of the two differing points in time is obtained by a linear interpolation between two differing values which 1are in the vicinity of a threshold value.

Embodiment 22: The method according to the pre-pre-preceding embodiment, wherein two differing values for the two differing points in time are used, wherein each of the two differing values are in the vicinity of a threshold value.

Embodiment 23: The method according to any one of the three preceding embodiments, wherein the two differing threshold values are selected from a range from -10 %/s to -0.1 %/s.

Embodiment 24: The method according to the preceding embodiment, wherein the two differing threshold values are selected from a range from -5 %/s to -2 %/s.

Embodiment 25: The method according to any one of the five preceding embodiments, wherein the two differing threshold values are selected according to a preliminary estimation of the body fluid concentration.

Embodiment 26: The method according to the preceding embodiment, wherein the body fluid comprises glucose, wherein the preliminary estimation of the body fluid concentration leads to a value of or above 100 mg/dl, and wherein the two differing threshold values selected are as -5 %/s and -2 %/s.

Embodiment 27: The method according to the pre-preceding embodiment, wherein the body fluid comprises glucose, wherein the preliminary estimation of the glucose concentration leads to a value below 100 mg/dl, and wherein the two differing threshold values selected are -2 %/s and -0.5 %/s.

Embodiment 28: The method according to any one of the preceding embodiments, wherein the body fluid comprises glucose, and wherein a hematocrit correction is applied to the glucose concentration.

Embodiment 29: The method according to the preceding embodiment, wherein the hematocrit correction is applied to the glucose concentration in case the hematocrit is outside a predetermined hematocrit range.

Embodiment 30: The method according to the preceding embodiment, wherein the predetermined hematocrit range comprises hematocrit values from 35 % to 50 %.

Embodiment 31: The method according to any one of the preceding embodiments, wherein, in step d), further the at least one disturbance variable Y is determined.

Embodiment 32: The method according to any one of the preceding embodiments, wherein the weighted average comprises weights which denote probabilities for each specific value of the at least one disturbance variable Y.

Embodiment 33: The method according to the preceding embodiment, wherein a forecast model provides a probability distribution of each specific value of the at least one disturbance variable Y.

Embodiment 34: The method according to any one of the preceding embodiments, wherein, in step b), a slope of the measurement curve is compared to at least one threshold value for determining if the measurement curve has reached the end value.

Embodiment 35: The method according to the preceding embodiment, wherein difference values of neighboring measurement values of the measurement curve are formed and compared to the at least one threshold value.

Embodiment 36: The method according to any one of the preceding embodiments, wherein, in step b), the end value is derived from at least one measurement value of the measurement curve and, in step c), the at least one second variable is derived from at least one fit parameter from the measurement curve.

Embodiment 37: The method according to any one of the preceding embodiments, wherein, in step b), the end value is derived from an earlier part of the measurement curve, wherein the earlier part is a part of the measurement curve being distant from a plateau of the measurement curve.

Embodiment 38: The method according to the preceding embodiment, wherein every measurement curve may form a same plateau value independent from the at least one disturbance variable Y.

Embodiment 39: The method according to any of the two preceding embodiments, wherein the end value may be determined from the same part of the measurement curve in which the decay constant $\Gamma$ or a quantity related to the decay constant $\Gamma$ may be determined as the second variable $x_2$.

Embodiment 40: The method according to any one of the preceding embodiments, wherein the evaluation part of the measurement curve is a remainder of the measurement curve starting after a definable starting time span after a commencement of a measurement.

Embodiment 41: The method according to the preceding embodiment, wherein the starting time span is a predetermined time span.

Embodiment 42: The method according to the preceding embodiment, wherein the predetermined time span is 0.5 s to 3 s, preferably 1.0 s to 2.0 s and most preferably 1.5 s to 1.7 s.

Embodiment 43: The method according to any one of the preceding embodiments, wherein the multivariate evaluation algorithm is determined by using a plurality of calibration measurements.

Embodiment 44: A computer program including computer-executable instructions for performing the method according to any one of the preceding embodiments when the program is executed on a computer or computer network.

Embodiment 45: An evaluation device for analyzing at least one sample of a body fluid, the evaluation device comprising at least one evaluation unit, wherein the evaluation unit is adapted to perform the method according to one of the preceding embodiments referring to a method for analyzing at least one sample of a body fluid.

Embodiment 46: A sample analysis device for analyzing a sample of a body fluid, the device comprising:

- at least one measuring unit for measuring a detection reaction of at least one test substance and at least one sample of a body fluid, wherein the detection reaction is known to be influenced by both a concentration c of an analyte to be detected in the body fluid and a set of disturbance variables, each disturbance variable characterizing at least one of a state of the sample of the body fluid and a condition of the detection reaction, the measuring unit further being adapted for monitoring a time development of at least one measurement value indicating a progress of the detection reaction, thereby recording a measurement curve F(t) containing a plurality of the measurement values acquired at different points in time, wherein at least an evaluation part of the measurement curve has an exponential characteristic; and
- at least one evaluation device according to the preceding embodiment.

Embodiment 47: The sample analysis device according to the preceding embodiment, furthermore comprising at least one test element, preferably at least one test strip, wherein the test element contains the at least one test substance adapted to perform the detection reaction, wherein the sample analysis device is adapted such that the sample of the body fluid is applicable to the test element.

Embodiment 48: The sample analysis device according to one of the two preceding embodiments, wherein the sample analysis device is embodied as a hand-held device.

Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.
In the Figures:

Figure 1          shows an exemplary embodiment of a sample analysis device according to the present invention in a cross-sectional view;

Figure 2A         shows measurement curves of a remission of a first test substance for two different glucose concentrations;

Figure 2B         shows first order derivatives of the measurement curves in Figure 2A;

Figures 3A and 3B  show exponential fits for the first order derivatives given in Figure 2B;

| Figures 4A and 4B | show an impact of the hematocrit on the fit parameters in the fit functions in Figures 3A and 3B; |
|---|---|
| Figures 5A and 5B | show fit functions of first order derivatives, in analogy to Figures 3A and 3B, with a different type of test substance; |
| Figure 6 | shows a remission curve used for subsequent evaluation in Figures 7A to 8B; |
| Figures 7A and 7B | show a first order derivative (Figure 7A) and second order derivative (Figure 7B) of the measurement curve in Figure 6; |
| Figure 8A | shows a quotient of the second order derivative and the first order derivative of Figures 7B and 7A; |
| Figure 8B | shows an exponential fit to the first order derivative in Figure 7A; |
| Figure 9 | shows an exemplary embodiment of a correlation between the end value EW or $x_1$, given as a relative remission rR in % and the glucose concentration c, for a hematocrit HKT 45; |
| Figure 10 | shows correction factors K to be applied to the correlation, as a function of the end value EW or $x_1$, given as a relative remission rR in % and as a function of the exponential fit parameter $\Gamma$ or $x_2$; and |
| Figures 11A and 11B | show residuals or deviations of the measured glucose concentration from the actual glucose concentration for uncorrected, univariate measurements (Figure 11A) and for corrected, multivariate measurements (Figure 11B). |
| Figure 12 | shows a first order derivative of a remission of a second test substance, a fit function of the first order derivative, and two different times $t_1$ and $t_2$ at two differing threshold values; |
| Figure 13 | shows decay rates for different hematocrit and glucose concentrations, wherein each decay constant $\Gamma$ is determined by two separate equations for the first derivative of equation (1) with neglected base line (a = 0) for two differing threshold values; |
| Figure 14A and 14B | show measured glucose concentrations determined by uncorrected, univariate values as average values over 10 measured values (Figure 14A) and as the corresponding measured values (Figure 14B); |
| Figure 15A and 15B | show measured glucose concentrations determined by corrected, multivariate values as average values over 10 measured values (Figure 15A) and as corresponding measured values (Figure 15B); |
| Figure 16 | shows a first decision tree, wherein a correction of the glucose values may only be applied outside a predetermined hematocrit range; |
| Figure 17 | shows a first order derivative of a remission of a second test substance, wherein the two different times $t_1$ and $t_2$ are determined by linear interpolation, whereas the time $t_1'$ is determined by the procedure as applied in Figure 12; |
| Figure 18 | shows a first order derivative of a remission of a second test substance, wherein the two different times $t_1$ and $t_2$ are selected from times at each of them actual values of the remission were acquired and each of them comprises the value of $R'(t_1)$ and $R'(t_2)$, respectively, i.e. the value of the first order derivative of the remission being closest to a predetermined threshold; |
| Figure 19 | shows a second decision tree, wherein, firstly, respective threshold values for determining the glucose concentration may be selected according to a predetermined glucose concentration range, and wherein, secondly, a correction of the glucose values may only be applied outside the predetermined hematocrit range; |

| Figure 20 | shows a third decision tree, wherein, first, depending on whether the glucose concentration may be within a predetermined glucose concentration range, the decay constant $\Gamma$ may be taken into account when determining the glucose concentration, and, secondly, depending on whether the decay constant $\Gamma$ may be equal to or exceed a predefined constant $\Gamma_0$, the hematocrit may be taken into account; and |

| Figure 21 | shows two typical measurement curves of the relative remission, wherein the two curves are distinguished from each other by their hematocrit, and two respective exponential fits for the corresponding hematocrit. |

Detailed description of the embodiments

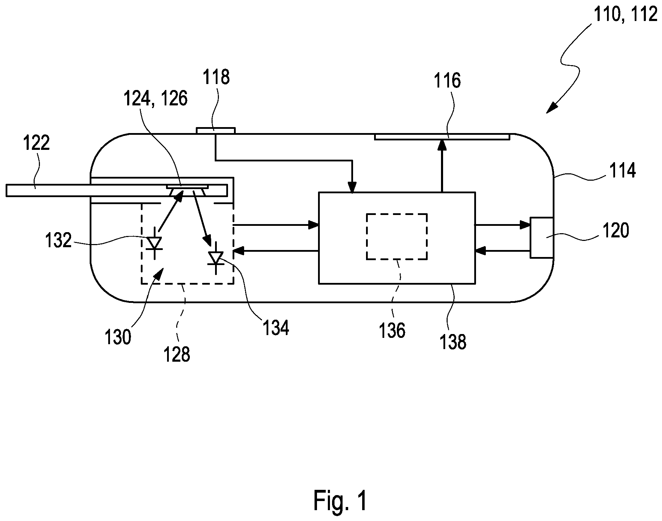

In Figure 1, an exemplary embodiment of a sample analysis device 110 according to the present invention is shown in a cross-sectional view, in a schematic setup. The sample analysis device preferably may be embodied as a hand-held device 112 and may comprise a casing 114 with one or more human machine-interfaces, such as one or more displays 116 and/or one or more controls 118, such as one or more push buttons and/or other types of controls. The sample analysis device 110 may further comprise one or more data interfaces 120, such as one or more infrared interfaces and/or wire-based interfaces and/or wireless interfaces. The sample analysis device 110 may further comprise an energy storage, such as a battery, which is not depicted.

The sample analysis device 110 is adapted for analyzing a sample of a body fluid applied to a test element 122. In the embodiment depicted in Figure 1, the test element 122 may be a strip-shaped test element, i.e. a test strip, having one or more test fields 124 to which the sample may directly or indirectly be applied. The test field 124 comprises a test substance 126 which is adapted to perform a detection reaction in the presence of an analyte, wherein the detection reaction is adapted to change at least one physical and/or chemical property of the test substance 126, which may be observed, preferably an optical characteristic. In the setup depicted in Figure 1, as an exemplary embodiment, the test substance 126 is adapted to change at least one optical property, such as a reflectance and/or a color.

For monitoring the progress of the detection reaction, the sample analysis device 110 comprises a measuring unit 128, which, in this exemplary embodiment, may comprise a detector 130 having at least one light source 132 for illuminating the test field 124, and further having at least one light-sensitive element 134 for detecting light reflected by the test field 124, preferably in an undirected manner, such as scattered light and/or diffused light. Thus, the detector 130 may be set up to perform a remission measurement on the test field 124. However, additionally or alternatively, other types of measurements for recording measurement curves containing a plurality of measurement values may be used.

The sample analysis device 110 further comprises an evaluation device 136, which may also function as a control device of the sample analysis device 110 and which may be connected to the display 116, the controls 118, the measuring unit 128 and the data interface 120, in a unidirectional and/or bidirectional manner. The evaluation device 136 may thus be adapted to control the overall functionality of the sample analysis device 110.

The evaluation device 136 comprises at least one evaluation unit 138, which may be or which may comprise a data processing device, such as a computer, preferably a microcomputer. The evaluation unit 138 is adapted to perform the method according to the present invention, as disclosed above or as will be disclosed in further detail below. For this purpose, the evaluation unit 138 may be adapted to initiate the acquisition of data by the measuring unit 128, such as the recording of the measurement curve, and/or may be adapted for performing the evaluation algorithm as disclosed above or as will be disclosed in further detail below.

It shall be noted that the sample analysis device 110 as depicted in Figure 1 is just one of many examples of analysis devices 110 adapted for performing the method according to the present invention.

As outlined above, the test element 122 comprises at least one test field 124 having at least one test substance 126. For the purpose of the exemplary embodiments of measurements and evaluation of these measurements as given below, two different types of test substance 126 were used:

As a first example of a test substance, in the following also referred to as the "PQQ chemistry", the test substance as disclosed in EP 0 354 441 A2 was used. This test substance comprises a PQQ-dependent dehydrogenase and a direct electron acceptor which is an aromatic nitroso compound or an oxim. This PQQ chemistry further comprises an optical indicator substance, i.e. a dye. As an example, hetero-polyblue indicator may be used, as disclosed in EP 0 431 456 A1.

As a second example of a test substance 126, in the following also referred to as "cNAD chemistry", the test substance as disclosed in one or more of documents WO 2007/012494 A1, WO 2009/103540 A1, WO 2011/012269 A2, WO 2011/012270 A1 and WO 2011/012271 A2 was used. Therein, WO 2007/012494 A1 generally discloses cNAD derivatives. WO 2009/103540 A1 discloses a stabilized enzyme/coenzyme complex. WO 2011/012269 A2, WO 2011/012270 A1 and WO 2011/012271 A2 disclose the synthesis of cNAD and cNAD-derivatives and intermediate products or precursors.

By using the PQQ chemistry and the cNAD chemistry, the following measurements were performed.

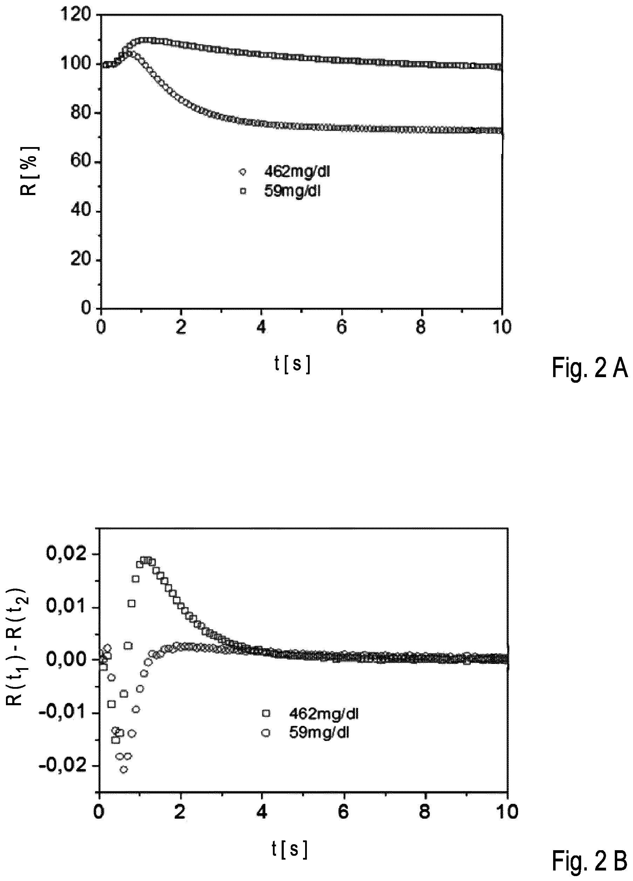

In a first set of measurements, depicted in Figures 2A to 5B, it was shown that, for both the PQQ chemistry and the cNAD chemistry, measurement curves of a remission characteristic may be recorded, such as by using the setup of Figure 1, which may very well be described by assuming an exponential characteristic of at least an evaluation part of the measurement curves. Thereby, in addition to an end value of the measurement curves, at least one fit parameter may be derived from the measurement curve and/or one or more derivatives of the measurement curve. Therein, the term fit parameter generally refers to a parameter which may be derived from the measurement curve itself and/or a first order or higher order derivative of the measurement curve.

Thus, in typical blood glucose measurements, the end value is used for determining the glucose concentration in blood. The determination of the end value, which may also be used within the present invention and which will not be explained in further detail in the following, may e.g. be performed according to EP 0 821 234 B1, US 2002/0146835 or EP 1 413 883 A1. Thus, as an example, the slope of the measurement curves may be compared to one or more threshold values and, as soon as the slope fulfills a predetermined condition, such as when the slope is below a given percentage per second (such as the remission curve having a negative slope of less than 2 % per second), the end value of the measurement curve may be determined.

In Figure 2A, measurement curves for two different blood glucose concentrations (462 mg/dl and 59 mg/dl) are shown. Therein, the relative remission R, as detected by detector 130, given in percent, is depicted as a function of measurement time t, given in seconds after sample application to the test element 122.

By using the end value algorithm, a first variable $x_1$ may be derived from the measurement curves in Figure 2A, which, in this measurement, may be determined to be approximately 73 % for the lower measurement curve (concentration c = 462 mg/dl) and approximately 100 % for the upper measurement curve (concentration c = 59 mg/dl).

Thus, in traditional measurements, one data value of the remission curves is used for determining the glucose concentration.

By using only the first variable $x_1$, i.e. the end value, the measurement results are highly susceptible to disturbances by one or more disturbance variables inherent to the sample and/or inherent to the measurement setup or the conditions of the measurement. Thus, as will be explained in further detail below, the hematocrit may have a significant impact on the glucose concentration as determined by the end value.

As disclosed above, the method according to the present invention therefore derives at least one further variable (second variable $x_2$) by taking into account an exponential characteristic of the measurement curve. For this purpose, the measurement curves themselves may be evaluated and/or one or more first order or higher order derivatives of the measurement curves, which, by themselves, form new measurement curves, may be used.

As an example, one or more of the fit functions (1) to (4) disclosed above may be used, wherein, in the following measurements shown in Figures 2A to 5B, the fit function (1) is used:

$$F(t) = a + b \cdot exp[-\Gamma t].$$

By using this fit function, the information of the measurement curve, i.e. of the remission kinetics, may be reduced to three parameters: the base line or offset a, the contrast or amplitude b, and the decay rate $\Gamma$. As will be shown, specifically the contrast b and the decay rate $\Gamma$ strongly depend on one or more disturbance variables, such as the hematocrit, the temperature or the relative humidity. Thus, by determining one or more of these fit parameters and using one or more of these fit parameters as a second variable $x_2$, in conjunction with an appropriate multivariate evaluation algorithm, a correction algorithm adapted for correcting the "raw glucose concentration" for the actual set of disturbance variables may be provided.

For performing an exponential fit, surprisingly, it turned out that a methodological advantage may be gained by using a first or higher order derivative of the measurement curves rather than the measurement curves themselves. In Figure 2B, first order derivatives of the measurement curves shown in Figure 2A are shown.

For generating the first order derivative or generating higher order derivatives, it turned out that these derivatives, in case the measurement curves are generated by using measurement values acquired at a constant acquisition frequency, may easily be derived by forming difference values of neighboring measurement values. Thus, in Figure 2B, difference values of neighboring measurement values are depicted as $R(t_1) - R(t_2)$. These differences are depicted as a function of the measurement time after sample acquisition. This type of analysis using first order or higher order derivatives of the measurement curve simplifies analysis, since e.g. the offset a should generally be eliminated, as depicted in Figure 2B.

It shall be noted, however, as already outlined in detail above, that other options are feasible. Thus, the measurement values not necessarily have to be acquired at a constant acquisition frequency. Preferably, however, the acquisition times and/or the time spans or time distances between neighboring measurement values are known, in order to derive the first order or higher order derivatives by dividing differences of neighboring measurement values by the respective time span between the measurement values, as known to the skilled person. The preferred option of using a constant acquisition frequency, however, allows for neglecting the aspect of the measurement time, since, in this case, the acquisition frequency simply provides a constant factor to all difference values between neighboring measurement values. Thereby, a significant simplification of the procedure may be achieved, which may lead to an increased speed of the algorithm and to a lowering of resources required for performing the algorithm.

It turned out that the curves depicted in Figure 2B may well be described by using an exponential characteristic, at least in an evaluation part of the measurement curve which starts at 1.7 s after sample application. In the following, as an evaluation part of the measurement curves, a time window of 1.7 s to 7 s after sample application to the test element 122 was used. The evaluation part of the measurement curve, however, may be optimized and may be adapted later on. Thus, the evaluation part may be adapted in case a different type of test substance 126 is used and may easily be determined for the measurement curves by appropriate tests.

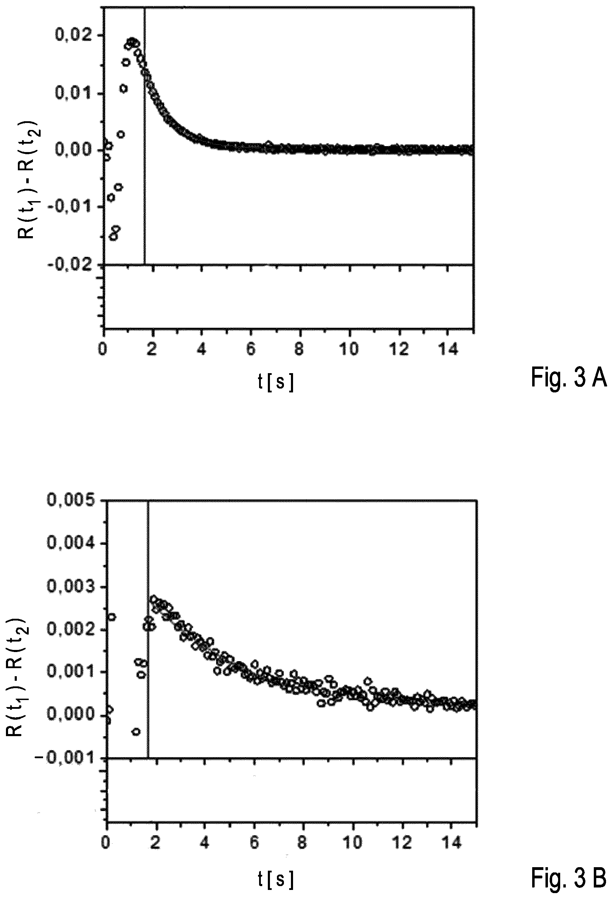

In Figures 3A and 3B, an exponential fit to the first order derivatives depicted in Figure 2B is shown. Therein, Figure 3A shows the first order derivative measurement curve for c = 462 mg/dl and Figure 3B shows the fit for c = 59 mg/dl. Therein, the solid lines depict the fit curves.

By using these fit functions, a contrast b of approximately 0.016 for c = 462 mg/dl and of b approximately 0.003 for 59 mg/dl was derived (both values given in percent), and a detail rate $\Gamma$ of approximately 0.93 1/s for 462 mg/dl and of approximately 0.22 1/s for 59 mg/dl.

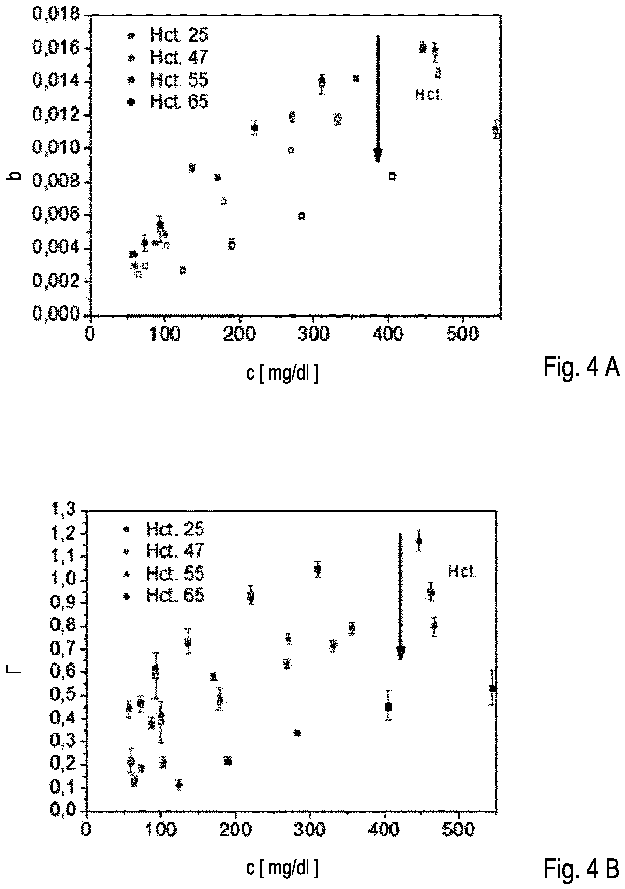

As it turned out, these fit parameters may strongly depend on one or more disturbance values, such as the temperature, the relative humidity or the hematocrit of the blood. This dependency is disclosed in Figures 4A and 4B. Therein, Figure 4A shows the influence of the hematocrit (Hct) on the contrast b, and Figure 4B shows the influence of the hematocrit on the decay rate $\Gamma$. In Figure 4A, the contrast b (given in percent) is depicted as a function of the concentration c, and in Figure 4B, the decay rate $\Gamma$, given in 1/s, is depicted as a function of the concentration c.

The measurement curves clearly show that, for one and the same glucose concentration c, the fit parameters b and $\Gamma$ significantly decrease with an increase in hematocrit. These measurements were performed by using a cNAD chemistry. Similar measurements may be performed for the influence of the relative humidity and show a similar dependency. In contrast with these results, it could be demonstrated that, at least under ambient conditions, the temperature may only slightly be able to influence these measurements as a kind of disturbance. However, other circumstances may be feasible. Consequently, the method according to the present invention may particularly be suited to be employed within a procedure of determining the glucose concentration c under the influence of the hematocrit and/or humidity.

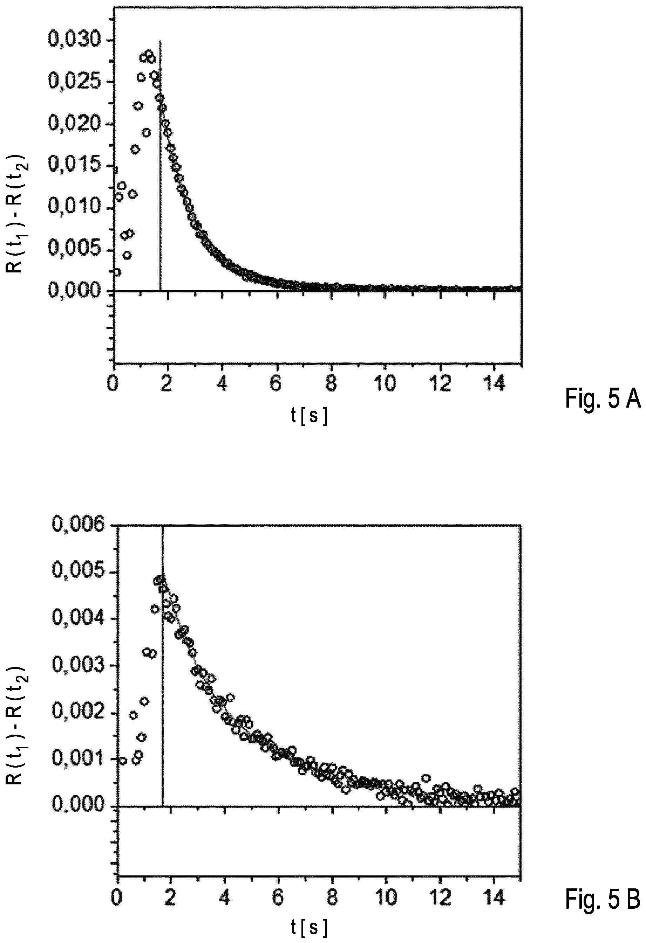

In Figures 5A and 5B, fit curves for PQQ chemistry, in analogy to the fit curves of Figures 3A and 3B, are shown, for concentrations of 462 mg/dl (Figure 5A) and 59 mg/dl (Figure 5B) which clearly demonstrates that the assumption of an exponential characteristic is valid for various types of test substances.

Thus, the measurements depicted in Figures 2A to 5B demonstrate that, at least in an evaluation region from 1.7 s after wetting to 7 s after wetting, the remission curves or their first order or higher order derivatives may well be described by assuming an exponential characteristic. Thereby, in addition to an end value, one or more further variables $x_2$ may be generated by generating appropriate fit parameters. These fit parameters and second variables depend on one or more disturbance variables, such as the hematocrit. Thus, by using the end value as a first variable $x_1$ and the at least one fit parameter as at least one second input variable $x_2$, a corrected value for the glucose concentration may be generated, by using an appropriate multivariate algorithm.

By the measurements shown in Figures 2A to 5B, two different concepts of the present invention were demonstrated: firstly, the option of evaluating the measurement curve itself, assuming an exponential characteristic of the measurement curve and, secondly, the option of using a first order or higher order derivative of the measurement curve as a "new measurement curve", for deriving the second variable $x_2$. In the following, two further concepts will be demonstrated, which may be used in addition or as an alternative.

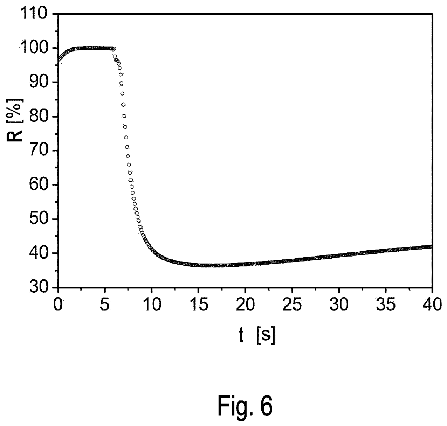

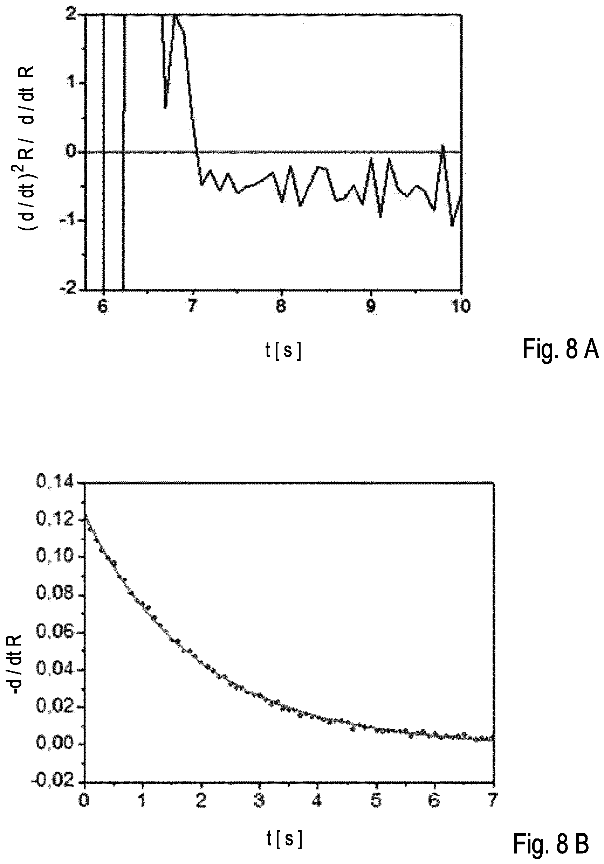

Thus, in Figures 6 to 8B, a third concept is demonstrated which is based on the use of two derivatives of higher order of the measurement curve as "new measurement curves". As an example, in Figure 6, a remission characteristic R is depicted as a function of time after application of the sample of the body fluid. This remission curve was derived by using the PQQ chemistry.

By assuming an exponential characteristic, such as the exponential characteristic (1) given above, it turns out that the decay rate $\Gamma$ of the exponential characteristic may be derived experimentally in a simplified manner. Thus, the derivative of $n^{th}$ order may be divided by the derivative of $(n-1)^{th}$ order, for n being an integer and $n \geq 1$. Thus, in case the base line is neglected (a = 0), as an example, the first order derivative is:

$$F'(t) = b \cdot \exp(-\Gamma t).$$

Similarly, the second order derivative may be calculated as:

$$F''(t) = -b \cdot \Gamma \cdot \exp(-\Gamma t).$$

By using these equations, the quotient of the second order derivative and the first order derivative is calculated as:

$$F''(t)/F'(t) = -b \cdot \Gamma \cdot \exp(-\Gamma t)/b \cdot \exp(-\Gamma t) = -\Gamma.$$

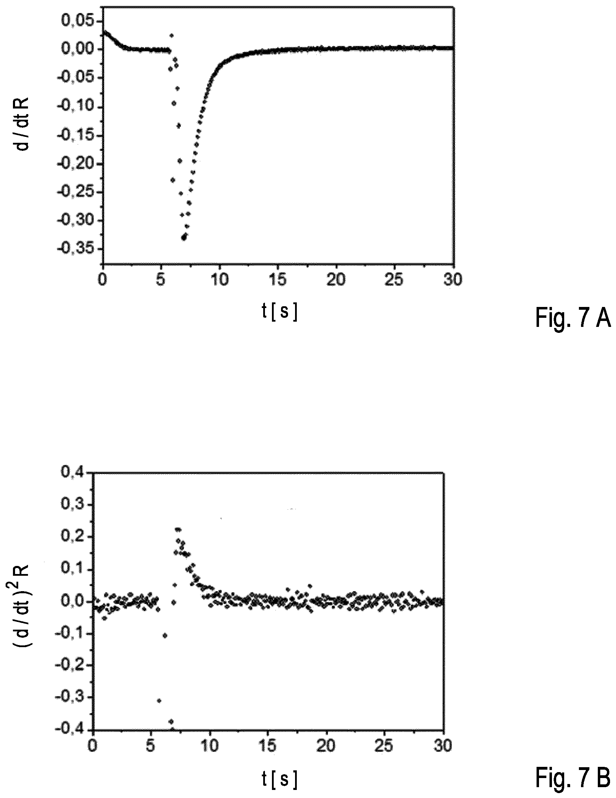

This idea allows for a simple and efficient evaluation of the measurement curves, as will be shown in Figures 7A to 8B. As an example, Figure 7A shows the first order derivative of the measurement curve, which may easily be generated by forming difference values between neighboring values, as disclosed with respect to Figure 2B above. Similarly, Figure 7B shows the second order derivative of the measurement curve, derived by forming difference values between neighboring measurement values of the first order measurement curve of Figure 7A. Higher order derivatives may be formed in a similar way.

In Figure 8A, a quotient of the measurement values of the measurement curves in Figures 7A and 7B is given as function of time t. As can be seen, the quotient, within uncertainty of measurement, starting at approximately 7 s, assumes a more or less constant value. For the first 20 values starting at 7.5 s, a mean value of $\Gamma$ = 0.494 1/s for concentrations of c = 136 mg/dl may be derived, and a value $\Gamma$ = 0.82441 1/s for glucose concentrations of 446 mg/dl. In Figure 8B, for reasons of comparison, an exponential fit to the first order derivative is depicted for concentrations of 136 mg/dl, which leads to a fit parameter $\Gamma$ of 0.507 1/s. The comparison of the measurements in Figures 8B and 8A clearly demonstrates that the fitting of an exponential curve may be replaced by a fit by using the quotient of two derivatives of the measurement curve of a different order. By both methods, the fit parameter $\Gamma$ may be derived which, by itself or in conjunction with other fit parameters, may be used as the at least one second variable, such as by using the multivariate evaluation algorithm given above. Thus, the quotient method as depicted in Figures 7A to 8B, specifically when generating derivatives by using the difference method disclosed above, leads to a simple and, still, effective fitting algorithm for deriving the fit parameter $\Gamma$ in a simple and effective way. Thereby, resources and time may be saved.

In addition to this third option (quotient method) as explained in conjunction with Figures 6 to 8B, other options for simplified generation of fit parameters exist. As an example of a fourth option, an integral may be used.

Thus, as an example, when the base line a is neglected (a = 0) or in case the first order or a higher order derivative of the measurement curve is used as a new measurement curve, the measurement curve may, as outlined above, be described by:

$$F'(t) = b \cdot \exp(\Gamma \cdot t)$$

wherein, as explained above, $\Gamma$ denotes the decay rate and b denotes the contrast. By integrating this function from 0 to oo, the following result may be derived:

$$\int_0^\infty b \cdot \exp(-\Gamma \cdot t) = b/\Gamma.$$

Thus, by using an integral and integrating over the measurement curve or a first order or higher order derivative of the measurement curve as a "new measurement curve", a simple and effective way of generating b/$\Gamma$ as a fit parameter and as a variable $x_2$ may be realized.

Similarly to the simplified method of forming a first order or higher order derivative of the measurement curve by using the difference method forming difference values of neighboring measurement values as disclosed above, the formation of an integral may be simplified, too. Thus, the integral may be calculated as:

$$\int_0^\infty b \cdot \exp(-\Gamma \cdot t) \approx \sum R_i \cdot \Delta t.$$

This approximation is also referred to as the Riemann integral or Riemann sum. Therein, the sum over the measurement values $R_i$ of the measurement curve or of the first order or higher order derivative of the measurement curve is formed over the evaluation part of the measurement curve. When assuming a constant measurement frequency, the time Δt between the measurement values is constant. In this case, the above-mentioned formula may be simplified to:

$$b/\Gamma \approx \Delta\text{t} \cdot \sum R_i,$$

wherein R; are the measurement values of the measurement curve or a first order or higher order measurement curve and wherein the sum is formed over the evaluation part of the measurement curve. As an example, for the measurements shown in Figures 2A to 3B, the sum may be formed from 1.7 s after wetting to 8.7 s after wetting. Thus, in a simple and efficient way, the fit parameter $b/\Gamma$ or similar fit parameters may be generated by using a simple integration process.

By using this integration, for a glucose concentration of 446 mg/dl, a value $b/\Gamma$ = 0.3164 was derived. This value, within experimental uncertainty, corresponds to the value $b/\Gamma$ = 0.2867 which was derived by fitting an exponential function to the first order derivative. For a glucose concentration of 136 mg/dl, by using the integration method, a value $b/\Gamma$ of 0.2353 was derived. By using an exponential fit, a value $b/\Gamma$ = 0.244 was derived.

As outlined above, the fit parameter $b/\Gamma$ may be used as the at least one second variable $x_2$ or as one of a plurality of second variables $x_2$ and, in combination with the first variable $x_1$, may be used in a multivariate evaluation algorithm, such as the algorithm disclosed above, for generating a corrected value of the glucose concentration, taking into account one or more disturbance variables, such as the hematocrit.

In addition to the one or more fit parameters derived by assuming an exponential characteristic, one or more of the disturbance variables which are known to have an impact on the evaluation of the glucose concentration or, generally, the analyte concentration, may be measured or detected independently. Thus, as an example, the temperature and/or the relative humidity may be measured independently. In this case, as an example, a plurality of multivariate evaluation algorithms may be provided, such as a set of evaluation algorithms, for the respective disturbance variables. Thus, as an example, one specific evaluation algorithm may be provided for a specific temperature and a specific relative humidity of the ambient atmosphere, wherein the multivariate evaluation algorithm for this specific temperature and relative humidity provides a corrected value for the glucose concentration, taking into account the end value of the remission curve as a first variable $x_1$ and the (unknown) hematocrit of the sample. For a different temperature and/or relative humidity, a different type of multivariate evaluation algorithm may be provided. Thus, a plurality of multivariate evaluation algorithms may be stored in the evaluation device 136 and/or the evaluation unit 138, which may contain a data storage device, and may be chosen in accordance with the measured values of the temperature and/or the relative humidity, for further use.

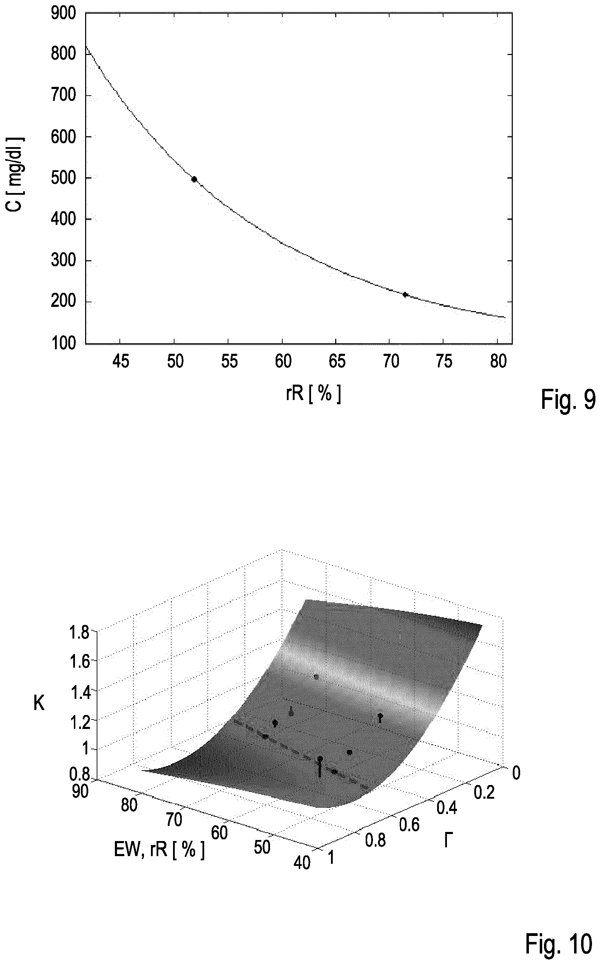

In order to demonstrate the power of the multivariate correction algorithm proposed by the present invention, in Figures 9 to 11B an exemplary embodiment of a correction algorithm is shown in detail. For these measurements, a cNAD-based test substance was used.

Therein, Figure 9 shows the actual glucose concentration c, given in milligrams per deciliter, as a function of the end value of the relative remission rR, also referred to as EW or $x_1$, given in %. Further, a polynomial fit function is shown. The actual glucose concentration is determined by a laboratory method, and the relative remission is measured by taking an optical measurement curve and determining the end value of this measurement curve. The hematocrit for these measurements was HKT=45.

As a basis for the fit function in Figure 9, a so-called code polynomial was used. This polynomial fit function is a univariate model which predicts the glucose concentration C as a function of the end value EW of the remission, in the following also referred to as y:

$$C(y) = c_1 + c_2 y + c_3 y^{b_1} + c_4 y^{b_2} + c_5 \exp(b_3 y)$$

In this formula, parameters $c_1,...,c_5$ und $b_1, b_2, b_3$ are free parameter is, which may be determined by using a calibration measurement, such as by using appropriate calibration fluid having known properties, such as a known hematocrit HKT45, a known glucose concentration and a known temperature. This calibration, also referred to as a generation of a code, typically is generated by using a set of data under standardized conditions, such as standard temperature, standard hematocrit (HKT45), standard humidity. Typically, more than two glucose concentrations are used for calibration, such as a plurality of glucose concentrations covering the whole sensible range of glucose concentrations which might occur in practical use.

By using this fit function, the following parameters were determined for the curve shown in Figure 9:

| Parameter | $c_1$ | $c_2$ | $c_3$ | $c_4$ | $c_5$ | $b_1$ | $b_2$ | $b_3$ |
|---|---|---|---|---|---|---|---|---|
| Value | -3,51 * 10^-4 | 10,3 | -6,21 * 10^5 | 0,508 | -2,29 * 10^-4 | -1,72 | 1,63 | 0,129 |

As outlined above, the measurement of Figure 9 was taken for one specific hematocrit HKT45. Thus, the algorithm is a univariate algorithm, deriving the glucose concentration from one variable, i.e. in this case the end value EW of the relative remission rR.

In order to derive a glucose concentration for an arbitrary hematocrit, the concentration c derived by the fit function formula of Figure 9 given above as to be corrected by a correction factor K, which itself may depend on the end value EW and the at least one exponential fit parameter, such as the exponential fit parameter $\Gamma$:

$$G = G(x_1, x_2) = G(EW, \Gamma)$$

$$= C(EW, HKT45) \cdot K(EW, \Gamma)$$

Again, the correction factor K may be separated into a term which is dependent on the end value EW ($=x_1$) of the glucose concentration and a term dependent on the at least one exponential fit parameter $\Gamma$ ($=x_2$), and it may be shown that the following fit formula may be applied:

$$K(EW, \Gamma) = (\Gamma^2 + a_1 \cdot \Gamma + a_2) \ / \ (c_1 \cdot EW^2 + c_2 \cdot EW + c_3)$$

This corresponds to a second end value - dependent correction of the first, $\Gamma$-dependent correction and, thus, to a multivariate correction algorithm comprising the end value EW as a first variable $x_1$ and the exponential fit parameter $\Gamma$ as a second variable $x_2$. The fit function comprises five independent parameters $a_1$, $a_2$ und $c_1$, $c_2$ und $c_3$. As a boundary condition, for HKT45, the correction factor shall be K=1, so the fit function of Figure 9 is obtained as a result.

By performing a plurality of calibration measurements for various hematocrits and by determining both the end value EW as a first variable $x_1$ and the at least one exponential fit parameter $\Gamma$ as a second variable $x_2$, a 3-dimensional calibration curve may be determined which is depicted for this exemplary embodiment in Fig. 10. Therein, the curved, shaded surface denotes the fit function of the correction factor K. As an example, for this specific embodiment, the following fit parameters of the above-mentioned equation were determined:

| Parameter | $f_1$ | $f_2$ | $a_1$ | $a_2$ |
|---|---|---|---|---|
| Value | -0,0049 | 0,8848 | -1,5580 | 1,2048 |

Thus, a corrected glucose concentration may be determined, by using the above-mentioned multivariate correction algorithm which both uses an end value of the measurement curve and at least one exponential fit parameter as input variables.

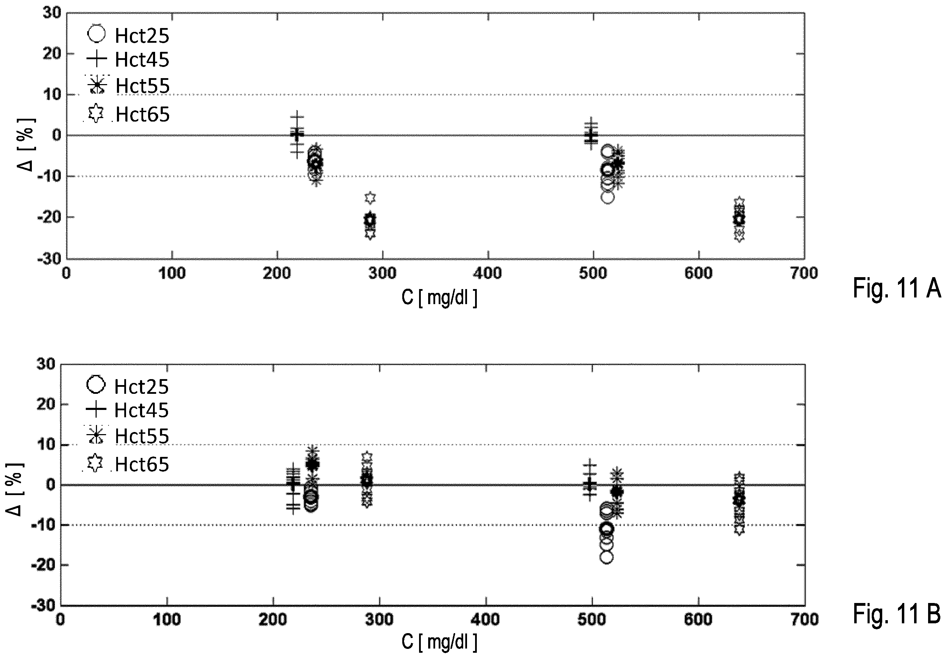

In Figures 11A and 11B, corrected and uncorrected glucose concentrations are depicted for the above-mentioned measurements of Figures 9 and 10. Therein, Figure 11A shows an uncorrected glucose concentration derived by using a univariate evaluation algorithm, based on the end value EW alone, as in Figure 9, which neglects the influence of the hematocrit HKT and which is based on the assumption of a hematocrit of HKT45. Contrarily, in Figure 11B, results of the method according to the present invention, using a multivariate algorithm, specifically using the correction algorithm disclosed above in conjunction with Figure 10, are shown. In each case, the deviation $\Delta$ is given for various actual glucose concentrations c, given in mg/dl, for various hematocrits. The actual glucose concentrations were determined by using a reliable laboratory method. The deviations are given in relative units [%].

As can be seen by comparing Figures 11A and 11B, the multivariate algorithm as proposed by the present invention significantly reduces the hematocrit-induced deviations. Thus, for hematocrits deviating from HKT45, the errors involved by evaluating the measurement curve and determining the glucose concentration thereof may widely be lower to a level of below 10% or 10 mg/dl. Thus, even though the algorithm may be kept rather simple, the accuracy of the measurement may be induced significantly.

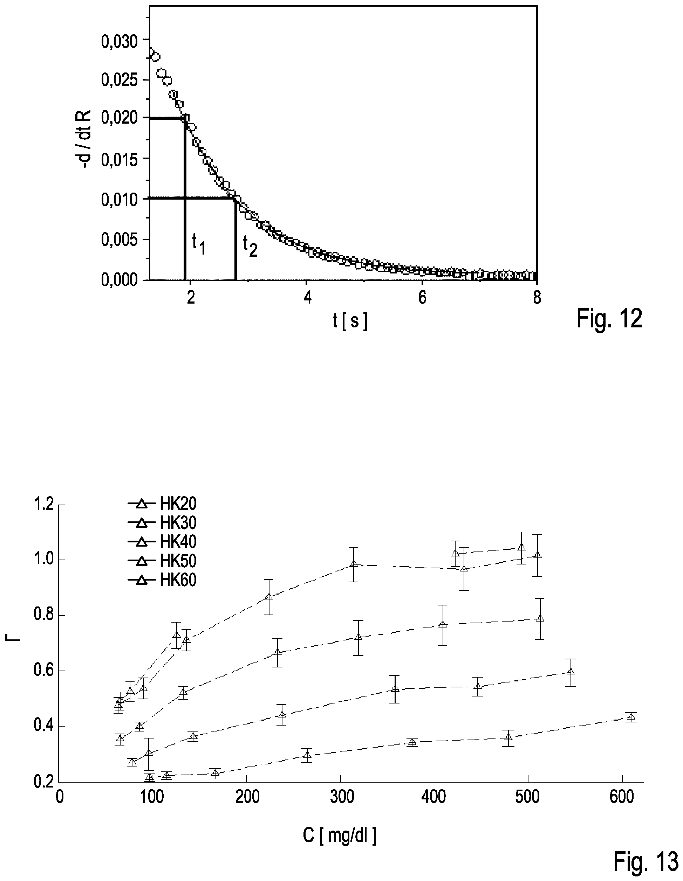

Figure 12 shows a first order derivative of a remission of a second test substance comprising a glucose concentration of c = 446 mg/dl, a hematocrit (Hct) of 25 %, a temperature of 23 °C, and a r. H. of 45 %. In addition, a fit function of the first order derivative, as well as two different times $t_1$ and $t_2$ at two differing threshold values are presented here.

The two different times $t_1$ and $t_2$ as depicted in Figure 12 may be determined by applying the first order derivative of the remission curve which exhibits an exponential characteristic. As an example, when the base line a is neglected (a = 0), the first order derivative of the remission curve may, as outlined above, be described by:

$$F'(t) = b \cdot \exp(\Gamma \cdot t)$$

Inserting a first threshold $F'(t_1)$ at a time $t_1$ in a first equation, and inserting a second threshold $F'(t_2)$ at a time $t_2$, will lead to the two following different equations:

$$F'(t_1) = b \cdot \exp(\Gamma \cdot t_1)$$

$$F'(t_2) = b \cdot \exp(\Gamma \cdot t_2).$$

Applying a rearranging of the two equations and a subsequent substitution, the following equation for the decay rate $\Gamma$ of the remission curve will be acquired:

$$\Gamma = \frac{\ln[F'(t_1) \, / \, F'(t_2)]}{[t_1 - t_2]}$$

As an example, inserting a first value of 2 %/s for the first threshold $F'(t_1)$ at a time $t_1$, and inserting a second value of 1 %/s for the second threshold $F'(t_2)$ at a time $t_2$, will lead to a value of the decay rate $\Gamma$ of the remission curve as follows:

$$\Gamma = \frac{\ln[0.01 / \, 0.02]}{[t_1 - t_2]}$$

Taking this example into account, it is evident that the determination of the decay rate $\Gamma$ of the remission curve may only require that the two different times $t_1$ and $t_2$ as, for example, depicted in Figure 12 are determined.

In the further course, this method has been applied to a set of 10 samples of whole blood, wherein each sample was adjusted to one of five different hematocrit concentrations, i.e. 20 %, 30 %, 40 %, 50 % or 60 %, as well as to one of seven different glucose concentrations within the range from 40 mg/dl to 600 mg/dl. Figure 13 shows various decay rates $\Gamma$ of the remission curve for the different hematocrit and glucose concentrations, wherein each decay rate $\Gamma$ is determined according to the method as described in connection with Figure 12, wherein a first value of -5 %/s for the first threshold $F'(t_1)$ at a time $t_1$, and a second value of -2 %/s for the second threshold $F'(t_2)$ at a time $t_2$ has been applied. Figure 13 clearly shows, on one hand, a strong dependence of the decay rate $\Gamma$ from the hematocrit and, on the other hand, a weak dependence from the glucose concentration. The mentioned values of -5 %/s for the first threshold and of -2 %/s for the second threshold may be applied with regard to a glucose concentration above 70 mg/dl.

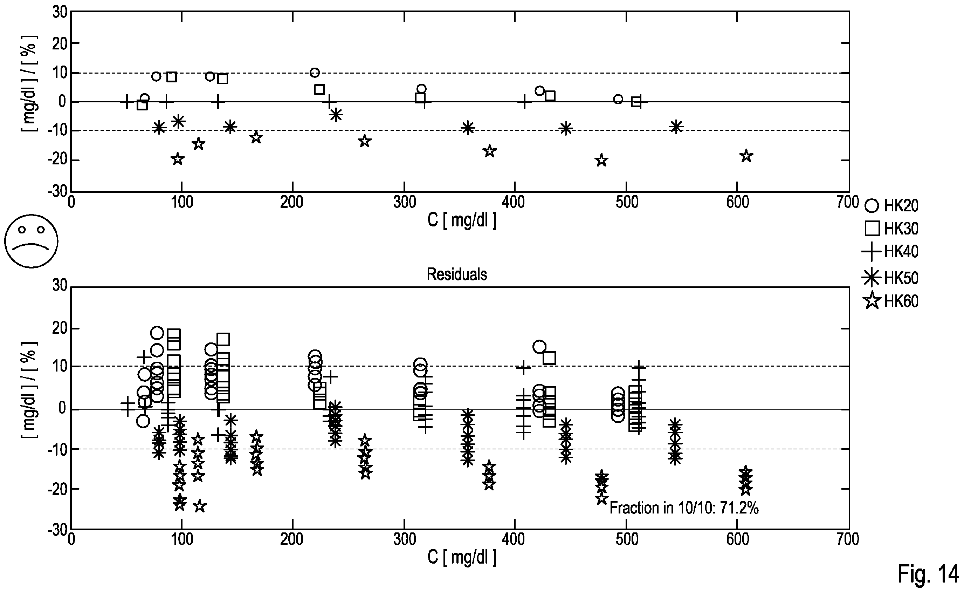

The method as exemplary described with respect to Figures 12 and 13 may allow determining a glucose concentration by applying a hematocrit correction with regard to the glucose concentration which may be acquired by using the respective threshold values. By determining glucose concentrations with a single threshold of -2 %/s for the remission decay a distribution as shown in Figures 14A and 14B will be obtained, wherein 71.2 % of all data points for the measured glucose values are distributed within a deviation of $\pm$10 % over the complete observed hematocrit range from 20 % to 60 %. Whereas Figure 14A shows measured glucose concentrations as average values over 10 measured values, Figure 14B depicts the corresponding single measured values. From Figure 14B it may be concluded that, particularly, samples with a hematocrit value of 60 % fall outside the desired range.

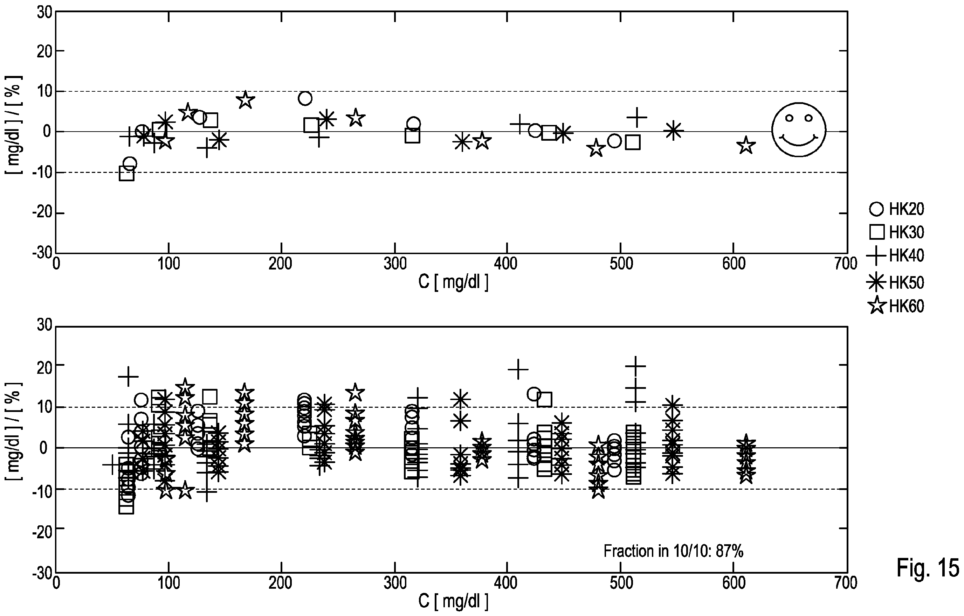

In contrast to the results as presented in Figures 14A and 14B, in the improved results as shown in Figures 15A and 15B, 87 % of all data points for the measured glucose values are distributed within a deviation of $\pm$10 % over the complete observed hematocrit range from 20 % to 60 %. This kind of improvement of more than 15 % with regard to the results from Figures 14A and 14B may be achieved by determining the glucose concentrations using a multivariate data analysis including the decay rate $\Gamma$ as determined above, for example, with the method as described in Figures 12 and 13. Whereas Figure 15A shows average values over 10 measured values, Figure 15B displays the corresponding single measured values.

However, it could have been observed that the coefficient of variation of all hematocrit values may increase when taking into account the hematocrit during the performance of the above mentioned measurements. Hereby, the coefficient of variation may be considered as a measure of a dispersion of a probability distribution of values which may be usually be defined as a ratio of the standard deviation to a mean value. This well-known effect may generally be observed during any hematocrit correction since no method is known so far by which the hematocrit may be determined exactly.

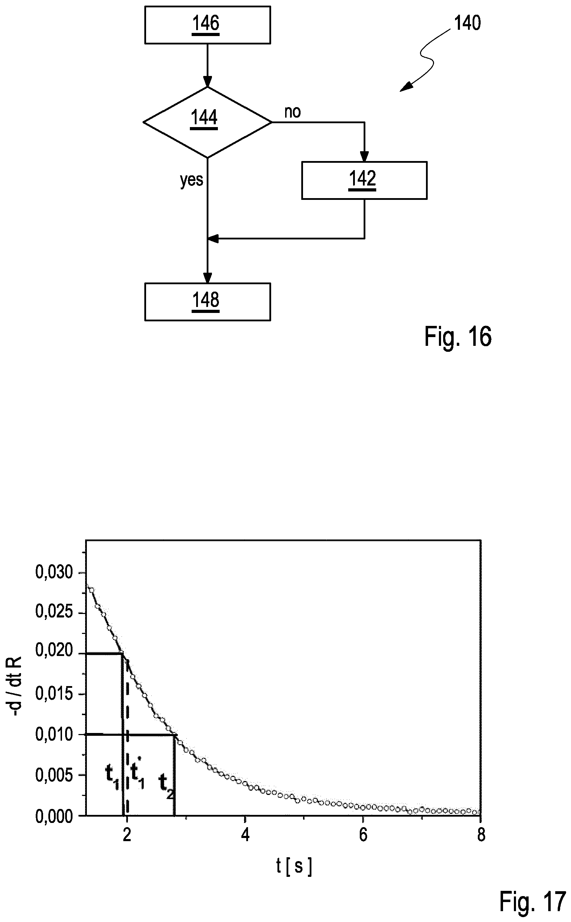

Preferentially, only such glucose values may be corrected for which such a correction may be required. As a preferred example, Figure 16 shows a first decision tree 140, wherein the hematocrit correction 142 of the glucose values may only be applied outside a predetermined hematocrit range 144. In particular, after a determination 146 of both the end value and the decay rate $\Gamma$, it may firstly be determined whether the hematocrit is inside or outside the predetermined hematocrit range 144, which preferably covers the range from 35 % to 50 %. However, other values for the predetermined hematocrit range 144 are possible. In this exemplary first decision tree 140, the hematocrit correction 142 of the glucose values may only be applied in case the hematocrit is outside the predetermined hematocrit range 144, here preferably covering the range from 35 % to 50 %. According to this discrimination, a determination 148 of a final value for the glucose concentration may be determined with or without hematocrit correction 142 depending on the actual value of the hematocrit.

Consequently, the first decision tree 140 as exemplary depicted in Figure 16 exhibits the positive effect that only such glucose values are submitted to the hematocrit correction 142 where the hematocrit correction 142 may be required for a further processing of the respective glucose values, in particular for rare cases in which a patient may display a very low or a very high hematocrit. Therefore, this kind of discrimination according to the first decision tree 140 may thus help to improve both the speed and the quality of the determination 148 of the final value of the glucose concentration under the influence of the hematocrit.

In Figure 17 a first order derivative curve of a remission curve is displayed, wherein the two different times $t_1$ and $t_2$ may be determined by linear interpolation of the corresponding data points before and after the respective first threshold $F'(t_1)$ at a time $t_1$ and the respective second threshold $F'(t_2)$ at a time $t_2$. This kind of procedure may be applied in order to determine the exact point in time at which the corresponding threshold will be achieved.

As an example, at the time $t_2$, the first order derivative curve may pass through the first order derivative of an actually measured value for the respective second threshold $F'(t_2)$. In contrast with this, no such first order derivative of a measured value may exist at the corresponding first threshold $F'(t_1)$ at the time $t_1$. In order to solve this problem, the time $t'_1$ may be determined according to the procedure as applied in Figure 12. However, according to Figure 17, a linear interpolation may be performed with regard to the first derivative of two actually measured values which are in the vicinity of the first threshold $F'(t_1)$ near the time $t_1$. This kind of procedure may be particularly useful in case of a high time resolution; otherwise it may be hard to approximate an exponential characteristic by a linear interpolation.

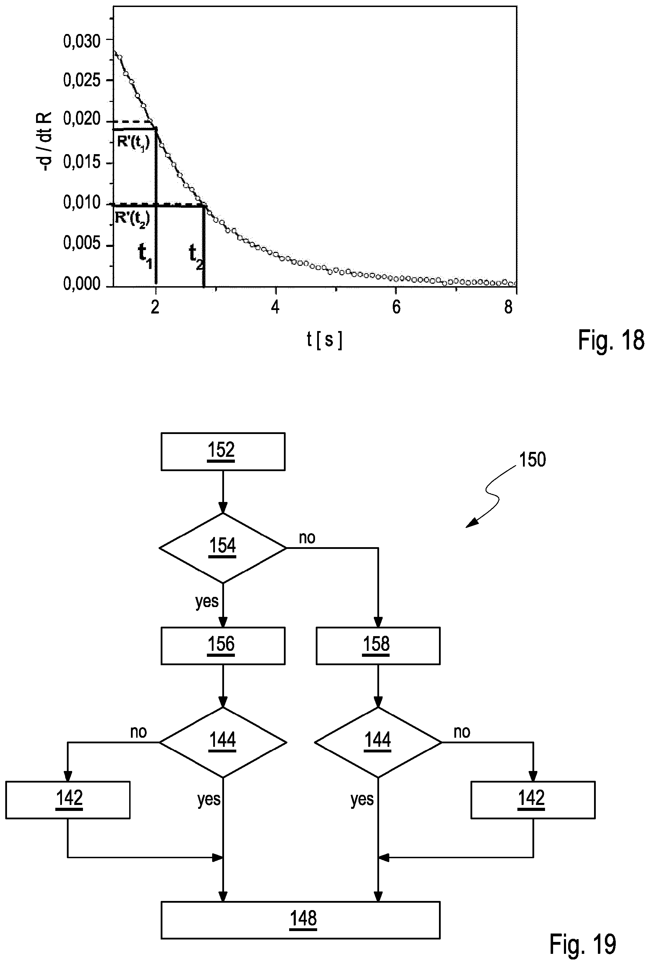

Figure 18 shows an alternative approach which may, in particular, be applied in a case of a low time resolution. Starting from the equation

$$\Gamma \; = \; \frac{\ln[F'(t_1) \, / \, F'(t_2)]}{[t_1 \, - \, t_2]} \, ,$$

actually determined values for a first threshold $F'(t_1)$ at a corresponding time $t_1$ as well as for the second threshold $F'(t_2)$ at a corresponding time $t_2$ are inserted into the equation, thus, leading to an exact value for the decay rate $\Gamma$. In a particularly preferred example, the values for the first threshold $F'(t_1)$ and the corresponding time $t_1$ as well as the values for the second threshold $F'(t_2)$ and the corresponding time $t_2$ are determined in a manner that both values for the threshold may be the values which are the closest to a predetermined threshold.

As decribed above, the decay rate $\Gamma$ could only be determined for glucose concentrations above 70 mg/dl. The reason for this observed behavior may be attributed to the fact that a first threshold value of -5 %/s has been applied within this kind of determination. The values of -5 %/s for the first threshold and of -2 %/s for the second threshold may be particularly applied since they seem to provide reasonable values for the decay rate $\Gamma$ over a large range of glucose concentrations. However, this way of procedure may not be applicable to a predetermined glucose concentration range which may be of 70 mg/dl or below since a glucose concentration within this range may not achieve the value of -5 %/s for the decay rate $\Gamma$.

Consequently, the determination 148 of the final value of the glucose concentration may be preferentially performed according to a second decision tree 150 as exemplary depicted in Figure 19. According to the second decision tree 150, the method may start with a determination 152 of the final value, from which a preliminary value for the glucose concentration may be derived. According to the fact whether the preliminary value for the glucose concentration falls within a predetermined glucose concentration range 152, firstly, respective first and second threshold values 156, 158 for determining the actual glucose concentration may be selected. In this example, in case the preliminary value for the glucose concentration may be estimated to be below 100 mg/dl, first and second threshold values 156 of -2 %/s for the first threshold and of -0.5 %/s for the second threshold may be particular-ly applied, whereas in case the preliminary value for the glucose concentration may be estimated to be 100 mg/dl or more, the above mentioned values of -5 %/s and of -2 %/s may be selected as first and second threshold values 158. However, other values the first threshold and for the second threshold may be chosen.

Secondly, in an additional second-order decision branch of the second decision tree 150, a hematocrit correction 142 of the glucose values may only be applied outside the predetermined hematocrit range 144. As already described above in relation to figure 16, the hematocrit correction 142 of the glucose values may only be performed in case the hematocrit takes a value outside a range of 35 % to 50 %, However, other values are possible. According to the discrimination as depicted in Figure 19, the determination 148 of a final value for the glucose concentration may be determined here also with or without hematocrit correction 142 depending on the actual value of the hematocrit. Hereby, the actual values chosen for the hematocrit correction 142 may be independent from the second-order decision branch of the second decision tree 150. Alternatively, for the hematocrit correction 142 actual values may be chosen which might depend on which second-order decision branch of the second decision tree 150 the hematocrit correction 142 may be performed.

Consequently, the second decision tree 150 as exemplary depicted in Figure 19 exhibits the positive effects that, firstly, very low glucose values even down to 40 mg/dl or below may be correctly determine, and that, secondly, only such glucose values are submitted to the hematocrit correction 142 where it may be required in particular for rare cases in which a patient may display a very low or a very high hematocrit. Therefore, this kind of discrimination according to the second decision tree 150 may thus help to improve both the speed and the quality of the determination 148 of the final value of the glucose concentration over a much larger range of glucose concentrations than before, thereby being able to taking into account the hematocrit for a correction of the glucose concentration.

In addition, a weighted average is employed within the method for analyzing the sample of the body fluid for taking into account a number of glucose concentrations measured on variations of the hematocrit, which may be considered as the disturbance variable Y, in order to derive the averaged concentration $\overline{c_{ave}}$ of the analyte:

$$\overline{c_{ave}} = \sum_{i=1}^{n} p_i \cdot c^i$$

or

$$\overline{c_{ave}} = \left( \sum_{i=1}^{n} p_i \cdot c^i \right) \Big/ \sum_{i=1}^{n} p_i$$

Herein, the weighted average $\overline{c_{ave}}$ may comprise weights $p_i$ which may denote probabilities for each specific value $c^i$ of the hematocrit according to a forecast model which may reflect the probability distribution of each specific value of the disturbance variable Y.

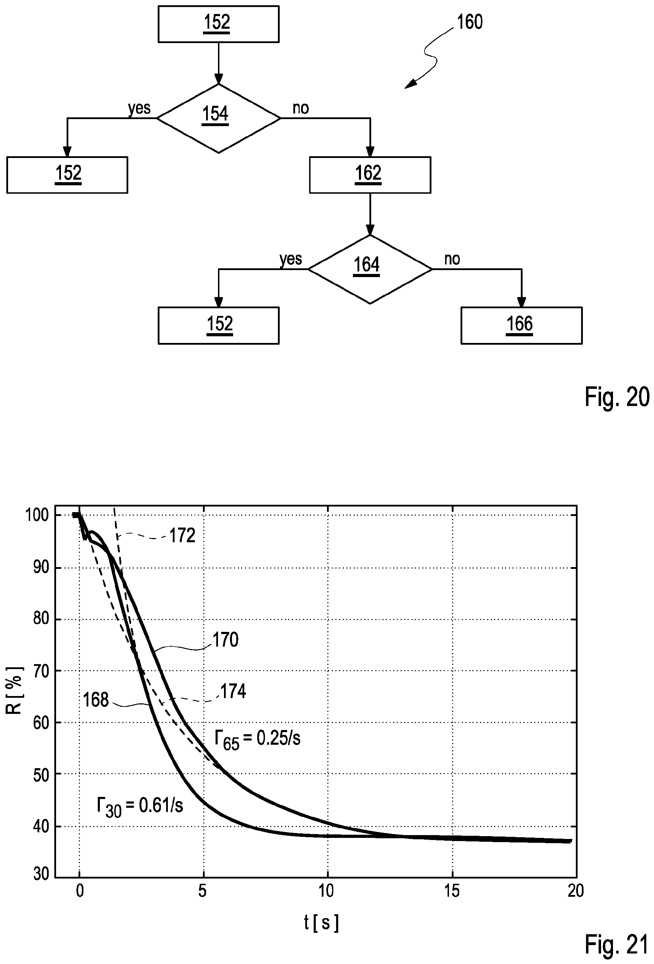

As a further example, Figure 20 shows a third decision tree 160, wherein from the determination 152 of the end value the preliminary value for the glucose concentration may be derived. According to an assessment whether the preliminary value for the glucose concentration may fall within the predetermined glucose concentration range 154, the preliminary value for the glucose concentration as acquired by the determination 152 of the end value may be kept or not. In the latter case, a determination 162 of the decay constant $\Gamma$ or the quantity related to the decay constant $\Gamma$, such as a quantity proportional to the decay constant $\Gamma$ or proportional to the inverse $1/\Gamma$ of the decay constant, may be performed. According to a further assessment 164 which might deliver an answer to the question whether the decay constant $\Gamma$ or the quantity related to the decay constant $\Gamma$ may be equal to or exceed a predefined constant $\Gamma_0$, the preliminary value for the glucose concentration as acquired by the determination 152 of the end value may still be kept or not. In the latter case, an additional evaluation procedure 166 for determining the glucose concentration may be performed, wherein the additional evaluation procedure 166 may take the hematocrit into account. Herein, the additional evaluation procedure 166 may further comprise another decision branch (not depicted here) which might branch out to different hematocrit evaluation procedures depending on whether the decay constant $\Gamma$ or the quantity related to the decay constant $\Gamma$ may be equal to or exceed a further predefined constant $\Gamma_1$. Thereby, a weighted average as described above is employed within at least one of the different hematocrit evaluation procedures.

This kind of decision tree, in particular the third decision tree 160 as schematically presented in Figure 20, may especially be employed for an evaluation of measurement curves as depicted in Figure 21. As a typical example, Figure 21 shows two measurement curves of the time dependence in s of the relative remission indicating the progress of the respective detection reactions of two blood samples each comprising a specific amount of glucose. Herein, both remission curves were derived by using a modified PQQ chemistry, wherein the usual PQQ chemistry was modified by employing an enzyme mutant. As can be depicted from Figure 20, the two remission curves are especially distinguished from each other by their respective amount of hematocrit. While a first measurement curve 168, being over most of the time, particularly within the evaluation part of the measurement curve, the lower curve, was recorded under a hematocrit of 30 %, a second measurement curve 170, being over most of the time, particularly within the evaluation part of the measurement curve, the upper curve, was recorded under a hematocrit of 65 %.

As further shown in Figure 21, both measurement curves 168, 170 could, particularly within the evaluation part of the measurement curve, be fitted by two respective exponential fits 172, 174. This feature particularly relates to the fact that the evaluation part of the measurement curve exhibits here an exponential characteristic and, by successfully allowing this kind of procedure, additionally proves this fact. Consequently, the first measurement curve 168 could, within the evaluation part of the measurement curve, be fitted by a first exponential fit 172, thereby providing a value of 0.61/s for the decay constant $\Gamma$, while the second measurement curve 168 could, also within the evaluation part of the measurement curve, be fitted by a second exponential fit 172, thereby providing a value of 0.25/s for the decay constant $\Gamma$. This example, as depicted in Figure 21, clearly demonstrates which kind of decisive impact the hematocrit may exert on the remission of blood samples, leading to a conclusion that, at least in some cases, inaccurate results for the glucose concentration may be acquired as long as the influence of the haematocrit might be not adequately taken in to account or even completely neglected. This situation which had been difficult to tackle so far may now be properly dealt with by applying the method according to the present invention.

List of reference numbers

[0152]

| | |
|---|---|
| 110 | sample analysis device |
| 112 | hand-held device |
| 114 | Casing |
| 116 | Display |
| 118 | Control |
| 120 | data interface |
| 122 | test element |
| 124 | test field |
| 126 | test substance |
| 128 | measuring unit |
| 130 | Detector |
| 132 | light source |
| 134 | light-sensitive element |
| 136 | evaluation device |
| 138 | evaluation unit |
| 140 | first decision tree |
| 142 | hematocrit correction |
| 144 | predetermined hematocrit range |
| 146 | determination of the end value and the decay rate |
| 148 | final determination of the value of the glucose concentration |
| 150 | second decision tree |
| 152 | determination of the end value |
| 154 | predetermined glucose concentration range |
| 156 | first and second threshold values determining the actual glucose concentration |
| 158 | first and second threshold values determining the actual glucose concentration |
| 160 | third decision tree |
| 162 | determination of decay constant |
| 164 | further assessment |
| 166 | additional evaluation procedure |
| 168 | first measurement curve |
| 170 | second measurement curve |
| 172 | first exponential fit |
| 174 | second exponential fit |

**Claims**

1.   A computer-implemented method for determining a concentration c of one or more analytes in at least one sample of a body fluid, thereby taking into account disturbances having an impact on a detection reaction, the method comprising the following steps:

a) recording a plurality of measurement values by monitoring a time development of at least one measurement value indicating a progress of a detection reaction of at least one test substance (126) and the sample of the body fluid, and providing at least one measurement curve $F(t)$ which contains the measurement values, wherein at least an evaluation part of the measurement curve has an exponential characteristic, wherein the measurement values contained in the measurement curve are acquired at differing points in time, wherein the detection reaction is known to be influenced by both a concentration c of an analyte to be detected in the body fluid and a set of disturbance variables Y, each disturbance variable characterizing at least one of a state of the sample of the body fluid and a condition of the detection reaction;

b) determining an end value of the measurement curve provided in step a), wherein the end value forms a first variable $x_1$, wherein the end value is determined from at least one measurement value of the measurement curve, for example, by monitoring or evaluating a slope or a change in the measurement curve, and, once the slope or change reaches a predetermined threshold, by determining an end point of the detection reaction, and by choosing some or more of the measurement values acquired at or after this end point as the end value and/or by deriving the end value by combining the measurement values, such as by forming a mean end value;

c) deriving at least one fit parameter from the measurement curve provided in step a) by taking into account the exponential characteristic of at least the evaluation part of the measurement curve, wherein the fit parameter forms at least one second variable $x_2$;

d) deriving the concentration c of the analyte by using at least one multivariate evaluation algorithm, the multivariate evaluation algorithm being adapted to combine the first variable $x_1$ provided by step b) and the second variable $x_2$ provided by step c) into a vector $(x_1, x_2)$, wherein a weighted average of the concentration c is derived from a result vector $(c, Y)$ of at least two procedures of the method and thereby provides the determined concentration, each procedure providing a result vector $(c, Y)$ via a matrix transformation from the vector $(x_1, x_2)$, comprising different $x_1$ and $x_2$ values for each procedure, wherein c is the concentration of one chosen analyte, each procedure being based on a variation of one type of disturbance variable Y.

2. The method according to any one of the preceding claims, wherein the disturbance variable Y comprises a parameter which is able to influence the viscosity of the body fluid.

3. The method according to any one of the preceding claims, wherein the at least one disturbance variable is selected from a particulate content of the sample, preferably a hematocrit.

4. The method according to any one of the preceding claims, wherein the exponential characteristic contains at least one exponential function selected from the group consisting of:

- $F(t) = a + b * \exp[-\Gamma^* t]$, wherein t is the time, a is an offset, b is a contrast and $\Gamma$ is a decay constant;
- $F(t) = a + b * \exp[-(\Gamma^* t)^\beta]$, wherein t is the time, a is an offset, b is a contrast, $\Gamma$ is a decay constant and $\beta$ is a stretching parameter.

5. The method according to any one of the preceding claims, wherein the second variable $x_2$ is selected from the decay constant $\Gamma$ or from a quantity which is in relationship with the decay constant $\Gamma$.

6. The method according to any one of the preceding claims, wherein, in step c), the fit parameter is obtained from a comparison of the first order derivative of the measurement curve at two differing points in time.

7. The method according to the preceding claim, wherein the two differing points in time are obtained by applying two differing threshold values.

8. The method according to the pre-preceding claim, wherein two differing values for the two differing points in time are used, wherein each of the two differing values are in the vicinity of a threshold value.

9. The method according to any one of the preceding claims, wherein, in step d), further the at least one disturbance variable Y is determined.

10. The method according to any one of the preceding claims, wherein the weighted average comprises weights which denote probabilities for each specific value of the at least one disturbance variable Y.

11. The method according to the preceding claim, wherein a forecast model provides a probability distribution of each specific value of the at least one disturbance variable Y.

12. The method according to any one of the preceding claims, wherein the multivariate evaluation algorithm is determined by using a plurality of calibration measurements.

13. A computer program comprising computer-executable instructions adapted to perform the method according to any one of the preceding claims when executed on a computer or computer network.

14. An evaluation device (136) for analyzing at least one sample of a body fluid, the evaluation device (136) comprising at least one evaluation unit (138), wherein the evaluation unit (138) is adapted to perform the method according to any one of the preceding method claims.

15. A sample analysis device (110) for analyzing a sample of a body fluid, the device comprising:

- at least one measuring unit (128) for measuring a detection reaction of at least one test substance (126) and at least one sample of a body fluid, wherein the detection reaction is known to be influenced by both a concentration c of an analyte to be detected in the body fluid and a set of disturbance variables, each disturbance variable characterizing at least one of a state of the sample of the body fluid and a condition of the detection reaction,
- the measuring unit (128) further being adapted for monitoring a time development of at least one measurement value indicating a progress of the detection reaction, thereby recording a measurement curve F(t) containing a plurality of the measurement values acquired at different points in time, wherein at least an evaluation part of the measurement curve has an exponential characteristic; and
- at least one evaluation device (136) according to the preceding claim.

**Patentansprüche**

1. Computerimplementiertes Verfahren zum Bestimmen einer Konzentration c von einem oder mehreren Analyten in mindestens einer Probe einer Körperflüssigkeit, wobei hierdurch Störungen berücksichtigt werden, die Auswirkungen auf eine Nachweisreaktion haben, wobei das Verfahren die folgenden Schritte umfasst:

a) Aufzeichnen einer Vielzahl von Messwerten durch Überwachen einer Zeitentwicklung von mindestens einem Messwert, die einen Fortschritt einer Nachweisreaktion von mindestens einer Testsubstanz (126) und der Probe der Körperflüssigkeit anzeigt, und Bereitstellen von mindestens einer Messkurve F(t), welche die Messwerte enthält, wobei mindestens ein Evaluierungsteil der Messkurve eine exponentielle Eigenschaft aufweist, wobei die in der Messkurve enthaltenen Messwerte zu verschiedenen Zeitpunkten erhalten wurden, wobei bekannt ist, dass die Nachweisreaktion von sowohl einer Konzentration c eines in der Körperflüssigkeit nachzuweisenden Analyten als auch einem Satz aus Störgrößen Y beeinflusst wird, wobei jede Störgröße mindestens eines von einem Zustand der Probe der Körperflüssigkeit und einer Bedingung der Nachweisreaktion kennzeichnet;
b) Bestimmen eines Endwerts der in Schritt a) bereitgestellten Messkurve, wobei der Endwert eine erste Variable $x_1$ bildet, wobei der Endwert aus mindestens einem Messwert der Messkurve bestimmt wird, zum Beispiel durch Überwachen oder Evaluieren eines Gefälles oder einer Änderung in der Messkurve, und, sobald das Gefälle oder die Änderung einen vorbestimmten Schwellenwert erreicht, durch Bestimmen eines Endpunkts der Nachweisreaktion, und durch Auswählen einiger oder mehrerer der an oder nach diesem Endpunkt erhaltenen Messwerte als dem Endwert und/oder durch Ableiten des Endwerts durch Kombinieren der Messwerte, etwa durch Bilden eines mittleren Endwerts;
c) Ableiten von mindestens einem Fitparameter aus der in Schritt a) bereitgestellten Messkurve durch Berücksichtigen der exponentiellen Eigenschaft von mindestens dem Evaluierungteil der Messkurve, wobei der Fitparameter mindestens eine zweite Variable $x_2$ bildet;
d) Ableiten der Konzentration c des Analyten mithilfe von mindestens einem multivariaten Evaluierungsalgorithmus, wobei der multivariate Evaluierungsalgorithmus dazu angepasst ist, die in Schritt b) bereitgestellte erste Variable $x_1$ und die in Schritt c) bereitgestellte zweite Variable $x_2$ zu einem Vektor $(x_1, x_2)$ zusammenzufassen, wobei ein gewichtetes Mittel der Konzentration c aus einem Ergebnisvektor (c, Y) aus mindestens zwei Durchgängen des Verfahrens abgeleitet wird und dadurch die bestimmte Konzentration liefert, wobei jeder Vorgang einen Ergebnisvektor (c, Y) über eine Matrixtransformation aus dem Vektor $(x_1, x_2)$ liefert, umfassend verschiedene $x_1$- und $x_2$-Werte für jeden Vorgang, wobei c die Konzentration eines gewählten Analyten ist, wobei jeder Vorgang auf einer Variation eines Typs der Störgröße Y basiert.

2. Verfahren nach einem der vorhergehenden Ansprüche, wobei die Störgröße Y einen Parameter umfasst, der die Viskosität der Körperflüssigkeit beeinflussen kann.

3. Verfahren nach einem der vorhergehenden Ansprüche, wobei die mindestens eine Störgröße aus einem Partikelgehalt der Probe, vorzugsweise einem Hämatokrit, ausgewählt ist.

4. Verfahren nach einem der vorhergehenden Ansprüche, wobei die exponentielle Eigenschaft mindestens eine exponentielle Funktion enthält, die ausgewählt ist aus der Gruppe bestehend aus:

- $F(t) = a + b * \exp[-\Gamma*t]$, worin t die Zeit ist, a ein Offset ist, b ein Kontrast ist und $\Gamma$ eine Zerfallskonstante ist;
- $F(t) = a + b * \exp[-(\Gamma*t)^\beta]$ , worin t die Zeit ist, a ein Offset ist, b ein Kontrast ist und $\Gamma$ eine Zerfallskonstante und ß ein Ausdehnungsparameter ist.

5. Verfahren nach einem der vorhergehenden Ansprüche, wobei die zweite Variable $x_2$ ausgewählt ist aus der Zerfallskonstante $\Gamma$ oder aus einer Größe, die in Beziehung zu der Zerfallskonstante $\Gamma$ steht.

6. Verfahren nach einem der vorhergehenden Ansprüche, wobei in Schritt c) der Fitparameter aus einem Vergleich der ersten Ableitung der Messkurve an zwei unterschiedlichen Zeitpunkten erhalten wird.

7. Verfahren nach dem vorhergehenden Anspruch, wobei die zwei unterschiedlichen Zeitpunkte durch Anwenden zweier unterschiedlicher Schwellenwerte erhalten werden.

8. Verfahren nach dem vorvorhergehenden Anspruch, wobei zwei unterschiedliche Werte für die zwei unterschiedlichen Zeitpunkte verwendet werden, wobei jeder der zwei unterschiedlichen Werte in der Nähe eines Schwellenwerts liegt.

9. Verfahren nach einem der vorhergehenden Ansprüche, wobei in Schritt d) ferner die mindestens eine Störgröße Y bestimmt wird.

10. Verfahren nach einem der vorhergehenden Ansprüche, wobei das gewichtete Mittel Gewichtungen umfasst, die Wahrscheinlichkeiten für jeden spezifischen Wert der mindestens einen Störgröße Y bezeichnen.

11. Verfahren nach dem vorhergehenden Anspruch, wobei ein Prognosemodell eine Wahrscheinlichkeitsverteilung eines jeden spezifischen Werts der mindestens einen Störgröße Y liefert.

12. Verfahren nach einem der vorhergehenden Ansprüche, wobei der multivariate Evaluierungsalgorithmus mithilfe einer Vielzahl von Kalibrierungsmessungen bestimmt wird.

13. Computerprogramm, das computerausführbare Anweisungen umfasst, die dazu angepasst sind, das Verfahren nach einem der vorhergehenden Ansprüche auszuführen, wenn auf einem Computer oder Computernetzwerk ausgeführt.

14. Evaluierungsvorrichtung (136) zum Analysieren von mindestens einer Probe einer Körperflüssigkeit, wobei die Evaluierungsvorrichtung (136) mindestens eine Evaluierungseinheit (138) umfasst, wobei die Evaluierungseinheit (138) dazu angepasst ist, das Verfahren nach einem der vorhergehenden Verfahrensansprüche durchzuführen.

15. Probenanalysevorrichtung (110) zum Analysieren einer Probe einer Körperflüssigkeit, wobei die Vorrichtung umfasst:

- mindestens eine Messeinheit (128) zum Messen einer Nachweisreaktion von mindestens einer Testsubstanz (126) und mindestens einer Probe einer Körperflüssigkeit, wobei bekannt ist, dass die Nachweisreaktion von sowohl einer Konzentration c eines in der Körperflüssigkeit nachzuweisenden Analyten als auch eines Satzes aus Störgrößen beeinflusst wird, wobei jede Störgröße mindestens eines von einem Zustand der Probe der Körperflüssigkeit und einer Bedingung der Nachweisreaktion kennzeichnet,
- die Messeinheit (128), welche ferner zum Überwachen einer Zeitentwicklung von mindestens einem Messwert angepasst ist, die einen Fortschritt der Nachweisreaktion anzeigt und dabei eine Messkurve F(t) aufzeichnet, die eine Vielzahl von Messwerten enthält, die zu verschiedenen Zeitpunkten erhalten wurden, wobei mindestens ein Evaluierungsteil der Messkurve eine exponentielle Eigenschaft aufweist; und
- mindestens eine Evaluierungsvorrichtung (136) nach dem vorhergehenden Anspruch.

**Revendications**

1. Procédé informatisé pour déterminer une concentration c d'une ou plusieurs substances à analyser dans au moins un échantillon d'un fluide corporel, prenant ainsi en compte les perturbations ayant un impact sur une réaction de détection, le procédé comprenant les étapes suivantes :

   a) enregistrement d'une pluralité de valeurs de mesure en surveillant l'évolution temporelle d'au moins une valeur de mesure indiquant une progression d'une réaction de détection d'au moins une substance de test (126) et de l'échantillon du fluide corporel et fournissant au moins une courbe de mesure $F(t)$ qui contient les valeurs de mesure, dans lequel au moins une partie d'évaluation de la courbe de mesure a une caractéristique exponentielle, dans lequel les valeurs de mesure contenues dans la courbe de mesure sont acquises à des moments différents, dans lequel il est connu que la réaction de détection peut être influencée à la fois par une concentration c d'une substance à analyser à détecter dans le fluide corporel et par un ensemble de variables de perturbation Y, chaque variable de perturbation caractérisant au moins l'un parmi un état de l'échantillon du fluide corporel et une condition de la réaction de détection ;
   b) détermination d'une valeur finale de la courbe de mesure fournie à l'étape a), dans lequel la valeur finale constitue une première variable $x_1$, dans lequel la valeur finale est déterminée à partir d'au moins une valeur de mesure de la courbe de mesure, par exemple en surveillant ou en évaluant une pente ou une modification de la courbe de mesure et, une fois que la pente ou la modification atteint un seuil prédéterminé, en déterminant un point final de la réaction de détection et en choisissant certaines des valeurs de mesure acquises sur ou après ce point final ou plus en tant que valeur finale et/ou en déduisant la valeur finale par une combinaison des valeurs de mesure, par exemple en formant une valeur finale moyenne ;
   c) obtention d'au moins un paramètre d'adaptation de la courbe de mesure fournie à l'étape a) en tenant compte de la caractéristique exponentielle d'au moins la partie d'évaluation de la courbe de mesure, dans lequel le paramètre d'adaptation constitue au moins une seconde variable $x_2$ ;
   d) obtention de la concentration c de la substance à analyser en utilisant au moins un algorithme d'évaluation à plusieurs variables, l'algorithme d'évaluation à plusieurs variables étant destiné à combiner la première variable $x_1$ fournie par l'étape b) et la seconde variable $x_2$ fournie par l'étape c) dans un vecteur $(x_1, x_2)$, dans lequel une moyenne pondérée de la concentration c est obtenue à partir d'un vecteur de résultat $(c, Y)$ d'au moins deux procédures du procédé et fournit ainsi la concentration déterminée, chaque procédure fournissant un vecteur de résultat $(c, Y)$ par l'intermédiaire d'une transformation matricielle à partir du vecteur $(x_1, x_2)$, comprenant différentes valeurs $x_1$ et $x_2$ pour chaque procédure, dans lequel c est la concentration d'une substance à analyser choisie, chaque procédure étant basée sur une variation d'un type de variable de perturbation Y.

2. Procédé selon l'une quelconque des revendications précédentes, dans lequel la variable de perturbation Y comprend un paramètre capable d'influencer la viscosité du fluide corporel.

3. Procédé selon l'une quelconque des revendications précédentes, dans lequel la variable ou les variables de perturbation sont choisies parmi une teneur en particules de l'échantillon, de préférence un hématocrite.

4. Procédé selon l'une quelconque des revendications précédentes, dans lequel la caractéristique exponentielle contient au moins une fonction exponentielle choisie dans le groupe constitué par :

   - $F(t) = a + b * \exp[-\Gamma*t]$, où t est le temps, a est un décalage, b est un contraste et $\Gamma$ est une constante de décroissance ;
   - $F(t) = a + b * \exp[-(\Gamma*t)^\beta]$ , où t est le temps, a est un décalage, b est un contraste, $\Gamma$ est une constante de décroissance et $\beta$ est un paramètre d'extension.

5. Procédé selon l'une quelconque des revendications précédentes, dans lequel la seconde variable $x_2$ est choisie parmi la constante de décroissance $\Gamma$ ou une quantité qui est en relation avec la constante de décroissance $\Gamma$.

6. Procédé selon l'une quelconque des revendications précédentes, dans lequel, à l'étape c), le paramètre d'adaptation est obtenu à partir d'une comparaison de la dérivée de premier ordre de la courbe de mesure à deux moments différents.

7. Procédé selon la revendication précédente, dans lequel les deux moments différents sont obtenus en appliquant deux valeurs de seuil différentes.

8. Procédé selon la revendication précédant la revendication précédente, dans lequel deux valeurs différentes sont utilisées pour les deux moments différents, dans lequel chacune des deux valeurs différentes se trouve au voisinage d'une valeur de seuil.

9. Procédé selon l'une quelconque des revendications précédentes, dans lequel, à l'étape d), en outre la variable ou les variables de perturbation Y sont déterminées.

10. Procédé selon l'une quelconque des revendications précédentes, dans lequel la moyenne pondérée comprend des poids qui indiquent des probabilités pour chaque valeur spécifique de la variable ou des variables de perturbation Y.

11. Procédé selon la revendication précédente, dans lequel un modèle de prévision fournit une distribution de probabilité de chaque valeur spécifique de la variable ou des variables de perturbation Y.

12. Procédé selon l'une quelconque des revendications précédentes, dans lequel l'algorithme d'évaluation à plusieurs variables est déterminé en utilisant une pluralité de mesures d'étalonnage.

13. Programme informatique comprenant des instructions exécutables par ordinateur destinées à mettre en oeuvre le procédé selon l'une quelconque des revendications précédentes lorsqu'elles sont exécutées sur un ordinateur ou un réseau d'ordinateurs.

14. Dispositif d'évaluation (136) pour analyser au moins un échantillon d'un fluide corporel, le dispositif d'évaluation (136) comprenant au moins une unité d'évaluation (138), dans lequel l'unité d'évaluation (138) est destinée à exécuter le procédé selon l'une quelconque des revendications précédentes du procédé.

15. Dispositif d'analyse d'échantillons (110) pour analyser un échantillon d'un fluide corporel, le dispositif comprenant :

    - au moins une unité de mesure (128) pour mesurer une réaction de détection d'au moins une substance de test (126) et d'au moins un échantillon d'un fluide corporel, dans lequel il est connu que la réaction de détection peut être influencée à la fois par une concentration c d'une substance à analyser à détecter dans le fluide corporel et par un ensemble de variables de perturbation, chaque variable de perturbation caractérisant au moins l'un parmi un état de l'échantillon du fluide corporel et une condition de la réaction de détection,
    - l'unité de mesure (128) étant en outre destinée à surveiller un développement dans le temps d'au moins une valeur de mesure indiquant une progression de la réaction de détection, enregistrant ainsi une courbe de mesure F(t) contenant une pluralité des valeurs de mesure acquises à différents moments, dans lequel au moins une partie d'évaluation de la courbe de mesure a une caractéristique exponentielle ; et
    - au moins un dispositif d'évaluation (136) selon la revendication précédente.

Fig. 1

Fig. 2 A

Fig. 2 B

Fig. 3 A

Fig. 3 B

Fig. 4 A

Fig. 4 B

Fig. 5 A

Fig. 5 B

Fig. 6

Fig. 7 A

Fig. 7 B

Fig. 8 A

Fig. 8 B

Fig. 9

Fig. 10

Fig. 11 A

Fig. 11 B

Fig. 12

Fig. 13

Fig. 14

Fraction in 10/10: 87%

Fig. 15

Fig. 16

Fig. 17

Fig. 18

Fig. 19

Fig. 20

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010094426 A1
- WO 2010094427 A1
- WO 2007012494 A1
- WO 2009103540 A1
- WO 2011012269 A2
- WO 2011012270 A1
- WO 2011012271 A2
- EP 0354441 A2
- EP 0431456 A1
- EP 0302287 A2
- EP 0547710 A2
- EP 1593434 A2
- EP 0821234 A

- US 20020146835 A1
- EP 0821234 B1
- WO 2011061257 A1
- US 20080087819 A1
- WO 0125760 A1
- EP 1413883 A1
- WO 2006138226 A2
- WO 9918426 A1
- US 5420042 A
- WO 2011131490 A2
- US 2008070234 A1
- US 20020146835 A

**Non-patent literature cited in the description**

- **J. HOENES et al.** *The Technology Behind Glucose Meters: Test Strips, Diabetes Technology & Therapeutics,* 2008, vol. 10 (1), 10-26